(19) Europäisches Patentamt
European Patent Office
Office européen des brevets

(11) **EP 2 753 421 B1**

(12) **EUROPÄISCHE PATENTSCHRIFT**

(45) Veröffentlichungstag und Bekanntmachung des Hinweises auf die Patenterteilung:
**10.04.2019 Patentblatt 2019/15**

(21) Anmeldenummer: **12758455.5**

(22) Anmeldetag: **07.09.2012**

(51) Int Cl.:
**B01J 19/24** (2006.01)     **C07C 45/50** (2006.01)
**C07C 209/60** (2006.01)

(86) Internationale Anmeldenummer:
**PCT/EP2012/067513**

(87) Internationale Veröffentlichungsnummer:
**WO 2013/034690 (14.03.2013 Gazette 2013/11)**

(54) **STRAHLSCHLAUFENREAKTOR MIT NANOFILTRATION**

JET LOOP REACTOR HAVING NANOFILTRATION

RÉACTEUR À JET ET À BOUCLE DE CIRCULATION À NANOFILTRATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.09.2011 DE 102011082441**

(43) Veröffentlichungstag der Anmeldung:
**16.07.2014 Patentblatt 2014/29**

(73) Patentinhaber: **Evonik Degussa GmbH**
**45128 Essen (DE)**

(72) Erfinder:
• **HAMERS, Bart**
**5961 VG Horst (NL)**
• **FRIDAG, Dirk**
**45721 Haltern am See (DE)**
• **FRANKE, Robert**
**45772 Marl (DE)**
• **BECKER, Marc**
**44359 Dortmund (DE)**
• **PRISKE, Markus**
**40472 Düsseldorf (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**WO-A1-2004/037803     WO-A1-2010/023018**
**DE-A1- 19 857 409**

EP 2 753 421 B1

**Beschreibung**

Die Erfindung betrifft eine Vorrichtung für die kontinuierliche, homogenkatalytische Umsetzung einer Flüssigkeit mit einem Gas und gegebenenfalls einem weiteren Fluid, wobei die Vorrichtung mindestens einen Reaktor mit einem von einer Pumpe getriebenen, äußeren Flüssigkeitsumlauf umfasst, und wobei die Vorrichtung mindestens eine den Homogenkatalysator bevorzugt zurückhaltende Membrantrennstufe aufweist.

Bekannt ist eine Vorrichtung dieser Gattung aus Janssen, M., Wilting, J., Müller, C. and Vogt, D. (2010), Continuous Rhodium-Catalyzed Hydroformylation of 1-Octene with Polyhedral Oligomeric Silsesquioxanes (POSS) Enlarged Triphenylphosphine. Angewandte Chemie International Edition, 49: 7738-7741; doi: 10.1002/anie.201001926.

Eine Flüssigkeit ist ein im Wesentlichen inkompressibles, fließfähiges Medium. Ein Gas ist ein kompressibles, fließfähiges Medium. Ein Fluid ist eine Flüssigkeit oder ein Gas. Ein zweiphasiges Gemisch aus einer homogen verteilten flüssigen Phase und eine dispers darin verteilte Gasphase ist ebenfalls ein Fluid im Sinne dieser Erfindung. Aufgrund des Gasanteils sind derartige Fluide im geringen Umfang kompressibel.

Im Rahmen der vorliegenden Erfindung wird unter zugeführter Flüssigkeit ein Stoff oder Stoffgemisch verstanden, der/das in der Apparatur unter Reaktionsbedingungen im flüssigen Aggregatzustand vorliegt und mindestens einen Reaktanden aufweist. Unter Gas wird ein reines Gas oder ein Gasgemisch, das zumindest einen Reaktanden und gegebenenfalls ein Inertgas aufweist, verstanden. Ein Beispiel für ein Gas, das zwei Reaktanden aufweist, ist das aus Wasserstoff und Kohlenmonoxid bestehende Synthesegas, das beispielsweise bei Hydroformylierungen eingesetzt wird.

Bei einem Strahlschlaufenreaktor (engl.: jet loop reactor) im Sinne der Erfindung handelt es sich um eine Vorrichtung für die kontinuierliche Umsetzung einer Flüssigkeit und mindestens eines weiteren Fluids, bei der die Flüssigkeit unter Druck durch eine Düse in einen Reaktionsraum eintritt, diesen längs einer Hauptstromrichtung durchströmt, an dem gegenüber der Düse gelegenen Ende des Reaktionsraums umgelenkt wird, entgegen der Hauptstromrichtung zurückströmt und erneut in Hauptstromrichtung beschleunigt wird, sodass sich innerhalb des Reaktorraums ein interner Flüssigkeitskreislauf (Schlaufe) einstellt. Das zweite Fluid wird von der Flüssigkeitsströmung mitgerissen und reagiert auf dem Weg entlang der Schlaufe. Die Flüssigkeit dient mithin als Treibstrahlmedium. Zum Eintrag der kinetischen Energie in die Flüssigkeit ist dem Reaktionsraum ein äußerer Flüssigkeitsumlauf zugeordnet, in welchem ein Teil der Flüssigkeit außerhalb des Reaktionsraums im Kreislauf geführt wird. Innerhalb des äußeren Flüssigkeitsumlaufs ist eine Pumpe vorgesehen, welche dem Flüssigkeitsstrom die für die Etablierung der Schleifenströmung innerhalb des Reaktors notwendige kinetische Energie aufprägt. Die Düse wird entsprechend aus dem äußeren Kreislauf gespeist.

Eine gute Einführung in die Technik der Strahlschlaufenreaktoren bietet: P. Zehner, M. Krause: "Bubble Columns", Ullmann's Encyclopedia of Industrial Chemistry, Electronic Release, 7th ed., Chapter 4, Wiley-VCH, Weinheim [2005].

Bei der Hydroformylierung (auch Oxo-Reaktion genannt) werden Kohlenwasserstoffe mit olefinischen Doppelbindungen (Alkene) mit Synthesegas (Gasgemisch aus Wasserstoff und Kohlenmonoxid) zu Aldehyden und/oder Alkoholen umgesetzt.

Grundlegende Einführungen in die Hydroformylierung bieten: Falbe, Jürgen: New Syntheses with Carbon Monoxide. Springer Verlag 1980, Berlin, Heidelberg, New York und Pruett, Roy L.: Hydroformylation. Advances in Organometallic Chemistry Vol. 17, Pages 1-60, 1979.

Die Hydroformylierung dient zur Herstellung von höheren Aldehyden. Höhere Aldehyde, insbesondere solche mit 3 bis 25 Kohlenstoffatome, werden zum Beispiel als Synthesevorstufen, zur Herstellung von Carbonsäuren und als Riechstoffe genutzt.

Technisch werden sie oft durch katalytische Hydrierung in die entsprechenden Alkohole überführt, die wiederum für die Herstellung von Weichmachern und Detergentien dienen. Aufgrund der großtechnischen Bedeutung der Hydroformylierungsprodukte wird die Oxo-Reaktion im industriellen Maßstab durchgeführt.

In der großtechnischen Hydroformylierung werden heute phosphororganische Metall-Komplexkatalysatoren auf Cobalt- oder Rhodiumbasis verwendet. Die Katalysatoren werden homogen in der flüssigen Hydroformylierungsmischung gelöst. Im Rahmen der Ausscheidung des Zielprodukts (der Aldehyde) aus dem Hydroformylierungsgemisch ist auch der Homogenkatalysator aus dem Hydroformylierungsgemisch schonend abzutrennen, da der Komplexkatalysator vergleichsweise empfindlich auf Zustandsänderungen reagiert und seine Aktivität verlieren könnte.

Traditionell wird der Katalysator destillativ aus den Hydroformylierungsgemisch abgeschieden. Um die Gefahr der Desaktivierung zu senken und den Energieverbrauch des Prozesses zu senken gibt es in jüngerer Zeit Bestrebungen, den homogen gelösten Katalysator mit Hilfe von Membrantechnologie (Nanofiltration) aus dem Hydroformylierungsgemisch abzutrennen.

Die Grundlagen der Membrangestützten, organophilen Nanofiltration zur Abscheidung von homogen gelösten Katalysatorkomplexen aus Hydroformylierungsmischungen beschreiben Priske, M. et al.: Reaction integrated separation of homogeneous catalysts in the hydroformylation of higher olefins by means of organophilic nanofiltration. Journal of Membrane Science, Volume 360, Issues 1-2, 15 September 2010, Pages 77-83; doi:10.1016/j.memsci.2010.05.002.

Bei der Membranfiltration von Reaktorausträgen von Hydroformylierungen stellt das häufig im flüssigen Reaktoraustrag gelöste oder ungelöste Synthesegas eine Besonderheit dar: Die Hydroformylierung ist eine zweiphasige Reaktion, Wasserstoff und Kohlenmonoxid bilden die Gasphase, die Alkene, Aldehyde und Alkohole die flüssige Phase, in welcher der Katalysator feststofffrei gelöst ist. Entsprechend dem Lösungsgleichgewicht im Reaktor ist auch ein Teil des Synthesegases in der flüssigen Reaktorphase gelöst und wird mit dem Reaktoraustrag entnommen. Solange das Synthesegas in dem Reaktoraustrag während der Membranfiltration gelöst bleibt, ist die Membranfiltration insoweit unproblematisch. Falls der flüssige Reaktoraustag jedoch von einer Gasphase begleitet wird oder wenn bei Entspannung an der Membran eine Gasphase ausperlt, vermögen die Gasblasen die Membran mechanisch zu schädigen. Für Schäden durch Gasblasen sind insbesondere Polymer-Membranen anfällig.

Ein weiteres Problem durch ausgasendes Synthesegas ist der Verlust an Kohlenmonoxid: Insbesondere in der Rh-katalysierten Hydroformylierung übt der CO-Partialdruck einen entscheidenden Einfluss auf die Aktivität und Stabilität des Katalysatorkomplexes auf. Um bei der Membranabtrennung von homogen gelösten Komplexkatalysatoren aus dem Reaktionsaustrag einer Hydroformylierung den Verlust von Aktivität zu vermeiden, schlägt EP1931472B1 vor, an allen drei Anschlüssen der Membrantrennstufe (Feed, Retentat, Permeat) einen CO-Mindestpartialdruck sicherzustellen.

WO2010023018A1 zeigt zwei parallel miteinander verschaltete Strahlschlaufenreaktoren mit einem gemeinsamen äußeren Flüssigkeitsumlauf. Die Strahlschlaufenreaktoren werden bei der Hydroformylierung mit homogen gelösten Katalysatoren eingesetzt. Die Abscheidung des Katalysators wird nicht thematisiert.

Janssen, M., Wilting, J., Müller, C. and Vogt, D. (2010), Continuous Rhodium-Catalyzed Hydroformylation of 1-Octene with Polyhedral Oligomeric Silsesquioxanes (POSS) Enlarged Triphenylphosphine. Angewandte Chemie International Edition, 49: 7738-7741. doi: 10.1002/anie.201001926 beschreiben die Durchführung einer homogen katalysierten Hydroformylierung in einem speziellen Sprühnebelreaktor, welcher über zwei äußere, in einer Querstromkammer miteinander kontaktierte Flüssigkeitsumläufe verfügt. In einem ersten Umlauf wird der flüssige Reaktoraustrag mit darin gelöstem Synthesegas aus dem Reaktor abgezogen und mittels einer Flügelzellenpumpe zirkuliert. In einer Querstromkammer wird der Reaktoraustrag in zwei Teilströme geteilt:

Ein erster Teilstrom enthaltend den flüssigen Reaktoraustrag mit gelöstem Synthesegas als Synthesegas in Gasphase wird entlang des ersten Umlaufes zurück in den Reaktor geführt. Ein zweiter, rein flüssiger Teilstrom wird mittels einer Pumpe über eine keramische Membrantrennstufe gefördert. Dort wird das Zielprodukt als Permeat abgezogen, das katalysatorhaltige Retentat über den zweiten Umlaufstrang zurück zur Querstromkammer geleitet und dort mit dem ersten Flüssigkeitsumlauf vermengt. Der Vorteil dieser Vorrichtung ist darin zu sehen, dass der Reaktoraustrag innerhalb der Querstromkammer entgast wird und somit etwaig ausfallende Gasphasen im ersten Umlauf verbleiben. Die besonderen Strömungsverhältnisse der Querstromkammer begünstigen nämlich einen Abzug der Gasblasen in den Rücklauf des ersten Kreises. Der zweite Flüssigkeitsumlauf, in welchem die Membran angeordnet ist, bleibt mithin gasfrei (das bedeutet, $H_2$ und CO bleiben in der Flüssigkeit gelöst). Der Nachteil dieses Labor-Apparats ist jedoch sein vergleichsweise komplizierter Aufbau, der Bedarf an zwei Pumpen und der große strömungsdynamische Energieverlust in der Querstromkammer: Hydroformylierungen im industriellen Maßstab lassen sich in dieser Vorrichtung kaum wirtschaftlich durchführen.

In Hinblick auf diesen Stand der Technik liegt der vorliegenden Erfindung die Aufgabe zu Grunde, eine Vorrichtung anzugeben, welche es gestattet, homogenkatalytische gas/flüssigphasen-Reaktionen, insbesondere Hydroformylierungen, welche mit Membranabscheidung des Katalysators arbeiten, in industrierelevantem Maßstab wirtschaftlich durchzuführen.

Gelöst wird diese Aufgabe durch eine Vorrichtung nach Anspruch 1.

Gegenstand der Erfindung ist daher eine Vorrichtung für die kontinuierliche, homogenkatalytische Umsetzung einer Flüssigkeit mit einem Gas und gegebenenfalls einem weiteren Fluid, welche mindestens einen Strahlschlaufenreaktor mit einem von einer Pumpe getriebenen, äußeren Flüssigkeitsumlauf umfasst, welche mindestens eine den Homogenkatalysator bevorzugt zurückhaltende Membrantrennstufe aufweist und bei welcher Pumpe und Membrantrennstufe in demselben äußeren Flüssigkeitsumlauf angeordnet sind.

Die Erfindung beruht auf der Erkenntnis, dass sich ein Strahlschlaufenreaktor und eine Nanofiltration zu einer Vorrichtung kombinieren lassen, die eine wirtschaftliche Durchführung einer Hydroformylierung im großtechnischen Format erlaubt. Grundlegend für die Wirtschaftlichkeit des Verfahrens sind ein einfacher Aufbau des Verfahrens mit möglichst wenigen Anlagenkomponenten sowie eine deutliche Rückhaltung und Rezirkulierung des aktiven Katalysators in die Reaktion. Ein auf die wesentlichen Komponenten reduzierter Prozess ergibt sich durch die direkte Integration der Membranabtrennung in den äußeren Kreislauf des Jet-Loop Reaktors. Dies ermöglicht zudem eine Abtrennung des Katalysators und gegebenenfalls des freien Liganden unter Reaktionsbedingungen.

Ein Einsatz handelsüblicher Membranmodule für Reaktionsgemische mit gelösten und/oder ungelösten Gasanteilen ist nicht möglich, da diese nicht für eine ausreichende permeatseitige Gasabfuhr ausgelegt sind und je nach Menge des permeatseitig abzuführenden Gasvolumenstroms permeatseitig einen Gegendruck aufbauen, welcher die Flüssigpermeatleistung reduziert oder sogar zu einer Zerstörung der Membran führen kann. Es wurde gefunden, dass der Einsatz von Membranmodulen, die pro Quadratmeter aktive Membranfläche ein permeatseitiges freies Volumen von mehr als 0.3 Kubikdezimeter ($dm^3$, entspricht 1 Liter) für permeatseitig auftretende Gasströme notwendig sind. Je höher der permeatseitige Gasstrom ist, desto größer muss das freie permeatseitige Volumen sein. Überraschenderweise wurde zudem gefunden, dass die Rückhaltung des Katalysators, in spätestens auf der Permeatseite eine Gasphase beinhaltende Prozessströme, mit der Zunahme des permeatseitigen freien Volumens erhöht wird.

Eine besonders bevorzugte Weiterbildung der Erfindung sieht daher vor, dass in der Membrantrennstufe ein permeatseitig freies Volumen vorgesehen ist, welches pro $m^2$ Membranfläche mindestens 0.3 $dm^3$ beträgt.

Als permeatseitiges Volumen ist das sich permeatseitig senkrecht zur aktiven Membranoberfläche anschließende Volumen zu verstehen. Das permeatseitige freie Volumen ist der Teil des permeatseitigen Volumens, welcher - abgesehen von dem Permeat - nicht mit Materie gefüllt ist.

In einer bevorzugten Ausführungsform der Erfindung ist die Pumpe baulich zum dauerhaften Pumpen von Fluiden eingerichtet, welche flüssige und gasförmige Phasen enthalten. Besonders bevorzugt sollten Pumpen eingesetzt werden, die zudem auch einen geringen Feststoffanteil fördern können.

Für diesen Zweck eignen sich besonders Peripheralradpumpen. Diese können dauerhaft Fluidgemische fördern, die eine flüssige und eine gasförmige Phase enthalten. Geringe Feststoffanteile stellen kein Problem dar. Eine Peripheralradpumpe ist eine besondere Bauform einer Kreiselpumpe mit einem ringförmigen Peripheralkanal, in welchem der Pumpenläufer rotiert. Der Pumpenläufer ist meist als kreisförmige Scheibe mit einer Mehrzahl von auf der Scheibe radial erstreckenden Vorsprüngen ausgeführt. Die Vorsprünge verlaufen im Bereich des Peripheralkanals. Das Fluid wird durch einen Saugstutzen angesaugt und den ringförmigen Kanal geleitet, in welchem sich der Pumpenläufer dreht. Dieser besteht aus geraden Schaufeln, die das Fluid in eine Rotationsbewegung versetzen. In dem Peripheralkanal wird also die Bewegungsenergie von den Schaufeln auf das Medium übertragen, wodurch der Druck erhöht wird. Das Fluid verlässt die Peripheralradpumpe durch einen Ablaufstutzen, nachdem es den Peripheralkanal durchströmt hat. Peripheralradpumpen sind komerziell erhältlich bei K-ENGINEERING Mischtechnik und Maschinenbau, 26871 Papenburg (Germany) oder bei SPECK PUMPEN Verkaufsgesellschaft GmbH, 91231 Neunkirchen a. Sand (Germany).

Um eine Desaktivierung des Katalysators und eine Beschädigung der Membran durch Entgasung zu vermeiden sowie einen verbesserten Membranrückhalt zu erreichen, ist das Permeat der Membrantrennstufe kontrolliert zu entgasen. In Ergänzung zu dem wie vorstehend dimensionierten, permeatseitig freien Volumen ist eine permeatseitig stromabwärts hinter der Membrantrennstufe angeordnete Gasabfuhr vorzusehen. Eine derartige Gasabfuhr sollte wie folgt aufgebaut sein:

Herzstück der Gasabfuhr ist ein Ausgleichsbehälter, in welcher flüssige und gasförmige Phasen voneinander getrennt sind. Der zweiphasige Permeatstrom wird dem Behälter zugeführt damit sich Flüssigphase und Gasphase trennen können. An die Gasphase ist eine Druckhaltevorrichtung angebracht, welche Gas bis zum Erreichen des vorgegebenen Drucks abführt. Zudem ist am Behälter eine Druckgaszufuhr angebracht, welche es ermöglicht den vorgegebenen Gasdruck, etwa bei abnehmendem Flüssigkeitsstand im Behälter, konstant zu halten.

Die Auswahl des geeigneten Membranwerkstoffs ist in Hinblick auf den abzuscheidenden Katalysatorkomplex zu treffen: Da die Durchlässigkeit einer Membran für die verschiedenen Bestandteile des zu trennenden Feeds letztendlich eine Funktion der Zeit ist (die Membran ist nicht absolut undurchlässig für einen Katalysator, seine Durchtrittsgeschwindigkeit ist vielmehr im Vergleich zu den anderen Reaktionsteilnehmer deutlich langsamer) ist die Membran so auszuwählen, dass sie den abzuscheidenden Katalysatorkomplex bevorzugt zurückhält.

In dem erfindungsgemäßen Verfahren können Membranen eingesetzt werden, die auf Grund von ihren chemischen oder physikalischen Eigenschaften geeignet sind, phosphororganischen Metall-Komplexkatalysator und/oder freien Organophosphor-Ligand vorzugsweise in einem Maße von zumindest 50 % zurückzuhalten.

Entsprechende Membranen gehören zu der Klasse der Nanofiltrations-Membranen. Der Begriff Nanofiltration wird auf Membrantrennverfahren angewendet, welche eine Trenngrenze bzw. Molecuar weight cut-off (MWCO) im Bereich von 150 g/mol bis zu über 1 nm besitzen. Die Größe der Trenngrenze (engl.: "molecuar weight cut-off" - MWCO) gibt die Molekül- oder Partikelgröße einer Komponente an, die einen Membranrückhalt von 90% besitzt.

Eine übliche Bestimmungsmethode für die Trenngrenze ist in Y.H. See Toh, X.X. Loh, K. Li, A. Bismarck, A.G. Livingston, In search of a standard method for the characterisation of organic solvent nanofiltration membranes, J. Membr. Sci., 291 (2007) 120-125 angegeben.

Der Membranrückhalt $R_i$ berechnet sich aus der feedseitigen Konzentration der betrachteten Komponente i an der Membran $x_{iF}$ und der pemeatseitigen Konzentration der betrachteten Komponente i an der Membran $x_{iP}$ wie folgt:

$$R_i = 1 - x_{iP} / x_{iF}$$

Bevorzugt sollten die Membranen für den erfindungsgemäßen Einsatz einen MWCO von kleiner 1000 g/mol aufweisen.

Eine weitere Vorraussetzung für die Verwendbarkeit der Membran besteht darin, dass die Membran stabil gegenüber allen in der Reaktionsmischung vorhandenen Verbindungen, insbesondere gegenüber den Lösemitteln sein muss. Als stabil werden auch Membranen angesehen, welche - etwa durch Quellen des Membranpolymers hervorgerufen - eine zeitliche Veränderung des MWCO und/oder der Permeabilität erfahren, jedoch über die Betriebsdauer die Trennaufgabe erfüllen. Darüber hinaus sollte der Membranwerkstoff die Reaktionstemperatur ertragen. Membranmaterialien, die Reaktionstemperatur stabil und performant sind, gestatten den Verzicht einer aufwändigen Temperaturregelung.

Vorzugsweise werden Membranen eingesetzt, die eine trennaktive Schicht aus einem Material, ausgewählt aus Celluloseacetat, Cellulosetriacetat, Cellulosenitrat, regenerierter Cellulose, Polyimiden, Polyamiden, Polyetheretherketonen, sulfonierten Polyetheretherketonen, aromatischen Polyamiden, Polyamidimiden, Polybenzimidazolen, Polybenzimidazolone, Polyacrylnitril, Polyarylethersulfone, Polyester, Polycarbonaten, Polytetrafluorethylen, Polyvinylidenfluorid, Polypropylen, Polydimethylsiloxan, Silicone, Polyphosphazene, Polyphenylsulfide, Polybenzimidazole, 6.6 Nylon, Polysulfone, Polyaniline, Polyurethane, Acrylonitril/Glycidylmethacrylat (PANGMA), Polytrimethylsilylpropyne, Polymethylpentyne, Polyvinyltrimethylsilan, alpha-Aluminiumoxide, Titaniumoxide, gamma-Aluminiumoxide, Polyphenylenoxid, Siliciumoxide, Zirconiumoxide, mit Silanen hydrophobisierte keramische Membranen, wie sie in DE10308111 beschrieben sind, Polymere mit intrinsischer Mikroporösität (PIM) wie PIM-1 und anderen, wie sie z. B. in EP0781166 und in "Membranes" von I. Cabasso, Encyclopedia of Polymer Sience and Technlogy, John Wiley and Sons, New York, 1987, beschrieben werden, aufweisen. Die oben genannten Stoffe können insbesondere in der trennaktiven Schicht ggf. durch Zugabe von Hilfsstoffen vernetzt vorliegen oder als so genannte Mixed Matrix Membranes mit Füllstoffen wie z. B. Carbon Nano Tubes, Metal Organic Frameworks oder Hollow Spheres sowie Partikel von anorganischen Oxiden oder anorganische Fasern, wie z. B. Keramik- oder Glasfasern versehen sein.

Besonders bevorzugt werden Membranen eingesetzt, die als trennaktive Schicht eine Polymerschicht aus Polydimethylsiloxan, Polyimid, Polyamidimid, Acrylonitril/Glycidylmethacrylat (PANGMA), Polyamid oder Polyetheretherketon aufweisen, die aus Polymeren mit intrinsischer Mikroporosität (PIM) wie PIM-1 aufgebaut sind, oder wobei die trennaktive Schicht über eine hydrophobierte keramische Membran aufgebaut ist. Ganz besonders bevorzugt werden Membranen aus Silikonen oder Polyamidimid eingesetzt. Solche Membranen sind kommerziell erhältlich.

Neben den oben genannten Materialien können die Membranen weitere Materialien aufweisen. Insbesondere können die Membranen Stütz- oder Trägermaterialien aufweisen, auf die die trennaktive Schicht aufgebracht ist. Bei solchen Verbundmembranen liegt neben der eigentlichen Membrane noch ein Stützmaterial vor. Eine Auswahl von Stützmaterialien beschreibt EP0781166, auf welches explizit verwiesen wird.

Eine Auswahl von kommerziell erhältlicher Nanofiltrationsmembranen sind die MPF und Selro Serien von Koch Membrane Systems, Inc., unterschiedliche Typen von Solsep BV, die Starmem™ Serie von Grace/UOP, die DuraMem™ und PuraMem™ Serie von Evonik Industries AG, die Nano-Pro Serie von Bio-Pure Technology, die HITK-T1 von IKTS, sowie oNF-1, oNF-2 und NC-1 von der GMT Membrantechnik GmbH.

Nach einer weiteren bevorzugten Ausführungsform der Erfindung ist im äußeren Flüssigkeitsumlauf, insbesondere stromaufwärts vor der Membrantrennstufe, ein Wärmetauscher zum Heizen oder Kühlen des Feeds oder des Permeats der Membrantrennstufe angeordnet. Der Wärmetauscher dient in der Regel zum Eintrag von Wärmeenergie in das System bei endothermen Reaktionen. Bei exothermen Reaktionen wird die Reaktionswärme über den Wärmetauscher abgeführt.

Für die Membranabtrennung kann in Abhängigkeit der Reaktionstemperatur eine Platzierung vor oder nach dem Wärmetauscher im äußeren Flüssigkeitsumlauf vorteilhaft sein. Höhere Temperaturen führen in der Regel zu höheren Permeabilitäten können aber je nach Membranmaterial zu einer Reduzierung des Membranrückhaltes führen.

In einer weiteren bevorzugten Ausführungsform der Erfindung erstreckt sich in dem Strahlschlaufenreaktor ein rohrförmiger Reaktionsraum, in welchen eine Strahldüse zum Eindüsen der Flüssigkeit in den Reaktionsraum sowie ein Saugrohr zum Ansaugen des Gases gemeinsam münden, und an welchem ein von einer Prallplatte abgeschirmter Abzug für den äußeren Flüssigkeitsumlauf vorgesehen ist.

Die Strahldüse kann in dem sich vertikal erstreckenden Reaktor nach oben oder nach unten gerichtet sein. Die gemeinsame Mündung von Strahldüse und Saugrohr bewirkt eine intensive Vermischung der flüssigen und gasförmigen Reaktionskomponenten (Wasserpumpeneffekt). Das Gas kann über das Saugrohr von außen angesaugt werden oder aus einem Bereich innerhalb des Reaktionsraums, in welchem sich eine Gasglocke erstreckt. Der Abzug kann oben oder unten am Reaktor angeordnet sein.

Die Abschirmung des Abzugs durch die Prallplatte verringert das Eintragen von Gasblasen aus dem inneren Flüssigkeitsumlauf in den äußeren Flüssigkeitsumlauf.

Zur strömungsdynamischen Verbesserung kann mindestens ein Leitrohr, welches sich konzentrisch durch den Reaktionsraum erstreckt, vorgesehen werden. Dadurch wird die Vermischung der flüssigen und gasförmigen Phase intensiviert. Es können auch mehrere Leitrohre fluchtend hintereinander angeordnet sein. Durch das Leitrohr strömt dann die Reaktionsmischung in Hauptstromrichtung, wird am Ende des Leitrohrs umgelenkt und strömt außerhalb des Leitrohrs zurück. Das Leitrohr stellt eine bauliche Trennung der beiden Strömungsrichtungen der inneren Schlaufe dar.

Wenn Strahlschlaufenreaktor und Membrantrennstufe in einem gemeinsamen äußeren Flüssigkeitsumlauf angeordnet sind, bedingt dies, dass der Durchsatz durch Reaktor und Membran gleich sein muss. Aus apparativen Gründen kann die Durchsatzleistung von Reaktor und Membran aber unterschiedlich sein. Um dann dennoch einen Flüssigkeitsumlauf oder ideale Membranüberströmung zu bewerkstelligen, ist es möglich, den Apparat mit der niedrigen Durchflussleistung mit einer Bypassleitung auszustatten, welche eine Teilumfahrung des hydraulischen Hindernisses ermöglicht. Dementsprechend weist die Vorrichtung mindestens eine Bypassleitung auf, welche im äußeren Flüssigkeitsumlauf parallel zum Strahlschlaufenreaktor oder zur Membrantrennstufe angeordnet ist.

In einer weiteren bevorzugten Ausführungsform der Vorrichtung weist diese nicht nur einen Stahlschlaufenreaktor auf, sondern eine Vielzahl von parallel schaltbaren Strahlschlaufenreaktoren mit einem gemeinsamen äußeren Flüssigkeitsumlauf, wobei die Membrantrennstufe innerhalb des gemeinsamen äußeren Flüssigkeitsumlaufs angeordnet ist. Eine Mehrzahl von spezifisch kleiner dimensionierten Strahlschlaufenreaktoren erlaubt durch Zu- und Abschalten einzelner Reaktoren eine flexible Anpassung der Umsatzleistung der Vorrichtung an die Bedarfslage. Dies ermöglicht eine wirtschaftliche Ausnutzung der Vorrichtung bei wechselnder Nachfrage.

Die Membrantrennstufe kann dementsprechend auch parallelisiert ausgeführt werden: Durch Zu- und Abschalten von einzelnen, parallel geschalteten Membranmodulen kann die Gesamtmembranfläche der Membrantrennstufe flexibel an die Anlagenkapazität angepasst werden. Eine bevorzugte Weiterbildung der Erfindung ist daher durch eine Membrantrennstufe gekennzeichnet, welche eine Vielzahl von dergestalt parallel schaltbaren Membranen umfasst, dass die gesamte aktive Membranoberfläche der Membrantrennstufe durch Zu- und Abschalten der Membranen einstellbar ist.

Die erfindungsgemäße Vorrichtung eignet sich in hervorragender Weise zur homogenkatalytischen Umsetzung einer Flüssigkeit mit einem Gas und gegebenenfalls einem weiteren Fluid, bei welchem das Zielprodukt der Umsetzung mit dem Permeat der Membrantrennstufe aus dem Flüssigkeitsumlauf ausgetragen wird.

Gegenstand der Erfindung ist mithin auch ein Verfahren zur homogenkatalytischen Umsetzung einer Flüssigkeit mit einem Gas und gegebenenfalls einem weiteren Fluid, wobei die die Umsetzung in einer erfindungsgemäßen Vorrichtung durchgeführt wird, und wobei das Zielprodukt der Umsetzung mit dem Permeat der Membrantrennstufe aus dem Flüssigkeitsumlauf ausgetragen wird.

Insbesondere dann, wenn eine Membrantrennstufe verwendet wird, welche ein permeatseitig freies Volumen von mindestens 0.3 dm$^3$ pro m$^2$ Membranfläche verfügt, ist es möglich, einen Flüssigkeitsumlauf mit gasförmigem Anteil zu prozessieren. Dies ist bis zu einem Gasanteil von etwa 30 Vol% möglich. Bei derartig hohen Gasanteilen ist jedoch das permeatseitige freie Volumen deutlich größer zu wählen als 0.3 dm$^3$. Eine bevorzugte Weiterbildung des erfindungsgemäßen Verfahrens besteht mithin darin, dass der äußere Flüssigkeitsumlauf stromaufwärts vor der Membrantrennstufe ein Gemisch darstellt, welches eine flüssige Phase und eine darin dispers verteilte gasförmige Phase umfasst, wobei Volumenanteil der gasförmigen Phase zwischen null und dreißig Prozent beträgt.

Diese Reaktionen können zweiphasig (flüssig / gasförmig) oder dreiphasig (flüssig / flüssig / gasförmig bzw. flüssig / gasförmig / gasförmig) sein. Im Flüssigkeitsumlauf können auch geringe Feststoffanteile vorhanden sein.

Beispiele für die durchführbaren Reaktionen sind Oxidationen, Epoxidierungen, Hydroformylierungen, Hydroaminierungen, Hydroaminomethylierungen, Hydrocyanierungen, Hydrocarboxyalkylierung, Aminierungen, Ammonoxydation, Oximierungen, Hydrosilylierungen, Ethoxylierungen, Propoxylierungen, Carbonylierungen, Telomerisierungen, Methathesen, Suzuki-Couplings oder Hydrierungen.

Besonders bevorzugt ist die Vorrichtung zur Hydroformylierung, also zur Umsetzung von Verbindungen mit olefinischen Doppelbindungen mit Synthesegas zu Aldehyden und/oder Alkoholen geeignet.

Die Verwendung der beschriebenen Vorrichtung zur Durchführung der genannten Verfahren ist ebenfalls erfindungsgegenständlich.

Die erfindungsgemäße Vorrichtung kann unter anderem für die Umsetzung einer Flüssigkeit mit einem Gas eingesetzt werden, wobei sowohl das Gas als auch die Flüssigkeit mindestens einen Reaktanden aufweisen.

Die Reaktionsprodukte werden in der Flüssigphase mit dem Permeat ausgetragen.

In der erfindungsgemäßen Apparatur können Reaktionen im Druckbereich von 0.2 bis 40 MPa (absolut) und im Temperaturbereich von 0 bis 350 °C durchgeführt werden. Die Reaktion findet dabei bevorzugt an einem in der flüssigen Phase homogen gelösten Katalysator statt.

Bevorzugt werden in der erfindungsgemäßen Vorrichtung Reaktionen durchgeführt, bei denen der Katalysator mit dem flüssigen Einsatzstoff eingespeist wird und in der flüssigen Produkt/Edukt-Phase homogen gelöst ist, wie beispielsweise die Herstellung von Aldehyden und/oder Alkoholen durch Hydroformylierung von Verbindungen mit olefinischen Doppelbindungen in Gegenwart von Kobalt- oder Rhodiumcarbonylen mit oder ohne Zusatz von Phosphor enthaltenden Liganden.

Die Erfindung soll nun anhand von Ausführungsbeispielen näher erläutert werden. Hierfür zeigen:

Fig. 1:    erfindungsgemäße Vorrichtung mit einem Strahlschlaufenreaktor
Fig. 2:    erfindungsgemäße Vorrichtung mit mehreren Strahlschlaufenreaktoren
Fig. 3:    erfindungsgemäße Vorrichtung mit mehreren Bypass-Optionen;
Fig. 4:    Darstellung einer Entgasung;
Fig. 5:    Darstellung permeatseitig freies Volumen bei einer Membran mit Permeatplatzhalter;
Fig. 6:    Darstellung permeatseitig freies Volumen bei einer Membran ohne Permeatplatzhalter;
Fig. 7:    rohrförmiges Membranmodul, schematisch in Axialansicht;

Fig. 8:     rohrförmiges Membranmodul aus Figur 7 im Längsschnitt;

Fig. 9:     rohrförmiges Membranmodul umfassend ein Bündel rohrförmiger Membranen;

Fig. 10:    rohrförmiges Membranmodul umfassend ein Bündel rohrförmiger Membranen mit gemeinsamer Stützstruktur.

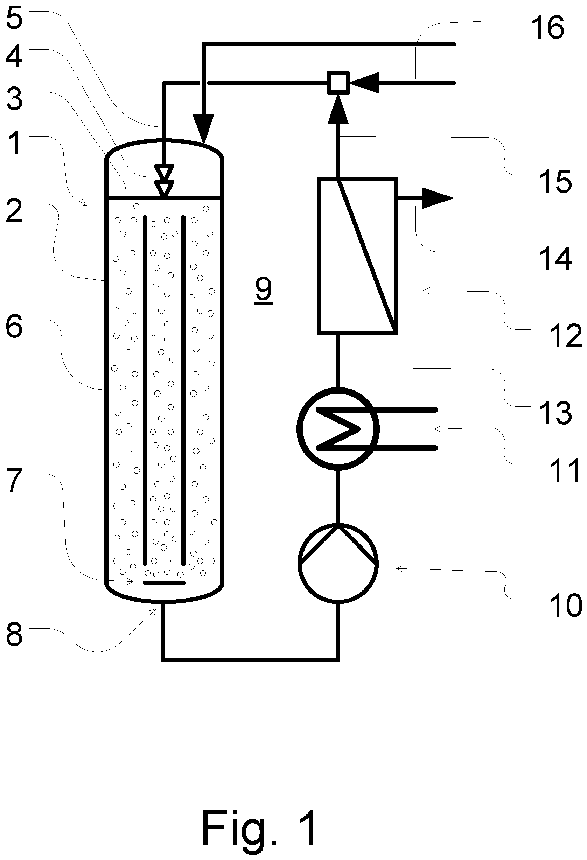

Figur 1 zeigt eine erste Ausführungsform der erfindungsgemäßen Vorrichtung mit einem Strahlschlaufenreaktor 1. Der Strahlschlaufenreaktor 1 umfasst einen rohrförmigen Reaktionsraum 2 in Gestalt eines Druckrohrs, der bis zu einem bestimmten Flüssigkeitsspiegel 3 mit flüssigem Reaktionsgemisch gefüllt ist. Oberhalb des Flüssigkeitsspiegels bildet sich eine Gasglocke aus gasförmige Reaktionsteilnehmer aus. Aufgrund des Lösungsgleichgewichts sind die gasförmigen Reaktionsteilnehmer teilweise im flüssigen Reaktionsgemisch gelöst, teilweise befinden sich gasförmige Reaktionsteilnehmer als Gasphase in der Flüssigkeit (in der Zeichnung als Gasblasen dargestellt). Ebenfalls in der Reaktionsflüssigkeit gelöst ist der Homogen-Katalysator.

In das flüssige Reaktionsgemisch hinein ragt von oben herab eine Strahldüse 4, über welche flüssige Reaktionsteilnehmer mit hoher kinetischer Energie eingedüst werden Gasförmige Reaktionsteilnehmer gelangen durch eine Gaszufuhr 5 in den Reaktionsraum 2. Der Stahldüse 4 baulich zugeordnet ist ein Saugrohr, welches das Gas aus dem mit Gas gefüllten Teil des Reaktionsraums 2 ansaugt und mit dem Fluidstrom vermischt. Hierzu sind die Mündungen von Saugrohr und Strahldüse eng benachbart und münden gemeinsam in den Reaktionsraum. Durch die hohe Strömungsgeschwindigkeit der aus der Strahldüse 4 austretenden flüssigen Reaktionsteilnehmer werden die gasförmigen Reaktionsteilnehmer mitgerissen (vergleichbar mit einer Wasserstrahlpumpe).

Durch den Reaktionsraum 2 erstreckt sich ein Leitrohr 6, konzentrisch und koaxial zum Druckrohr. Das Leitrohr 6 dient dazu, innerhalb des Reaktionsraums 2 einen inneren Flüssigkeitsumlauf zu schaffen: Die eingedüste Reaktionsflüssigkeit strömt von der Strahldüse 4 herab durch das Leitrohr 6 und wird von dem am anderen Ende des Reaktionsraums 2 angeordnete Prallplatte 7 umgelenkt, sodass der Strom außerhalb des Leitrohrs 6 wieder nach oben strömt. Auf diese Weise entsteht innerhalb des Reaktionsraums 2 ein innerer Flüssigkeitsumlauf, in welchem die Reaktionspartner intensiv vermischt und umgesetzt werden.

Unterhalb der Prallplatte 7 ist ein Abzug 8 vorgesehen, durch welchen kontinuierlich Reaktionsgemisch aus dem Reaktionsraum 2 abgezogen und in einen äußeren Flüssigkeitsumlauf 9 eingespeist wird. Die Prallplatte 7 schirmt den Abzug 8 zum inneren Flüssigkeitsumlauf ab, sodass Gasblasen kaum in den äußeren Flüssigkeitsumlauf 9 geraten. Der äußere Flüssigkeitskreislauf besteht daher überwiegend aus flüssigen Reaktanden, gelöstem Katalysator und gelösten gasförmigen Reaktanden.

Für die Ausübung der Erfindung ist es unerheblich, ob Strahldüse 4 nach unten gerichtet und Prallplatte 7 unterhalb der Strahldüse 4 angeordnet ist. Es ist auch möglich, vom Boden des Reaktors her nach oben einzudüsen. Der Abzug kann in beiden Fällen oben oder unten im Reaktor angeordnet sein. Die Prallplatte ist entsprechend so anzuordnen, dass sie den Abzug abschirmt.

Der äußere Flüssigkeitsumlauf 9 wird von einer Pumpe 10 bewegt. Bei der Pumpe 10 handelt es sich um eine Peripheralradpumpe, welche auch flüssig/gasförmige Gemische zu fördern vermag. Geringfügige Gasblasen sind daher unschädlich.

Stromabwärts hinter der Pumpe 10 ist ein Wärmetauscher 11 angeordnet, mittels welchem abhängig von dem Reaktionstyp Wärme in den äußeren Flüssigkeitsumlauf 9 ein- oder ausgetragen werden kann. Darüber hinaus kann der Strahlschlaufenreaktor 1 selbst mit einem Wärmetauscher versehen sein, welcher den Reaktionsraum umgibt (nicht dargestellt).

Stromabwärts hinter dem Wärmetauscher 11 ist eine Membrantrennstufe 12 angeordnet. Die Membrantrennstufe kann auch vor dem Wärmetauscher liegen. Wie jede Membran, weist die Membrantrennstufe 12 drei Anschlüsse auf, nämlich Feed 13, Permeat 14 und Retentat 15. Das über den Feed 13 anströmende Reaktionsgemisch wird an der Membran in Permeat 14 und Retentat 15 getrennt. Da die Membran für den gelösten Katalysatorkomplex weniger durchlässiger ist als für die übrigen Komponenten des Feeds, verbleibt der Katalysator diesseits der Membran und wird im Retentat 15 angereichert. Bezogen auf den Katalysator weist die Membran eine bessere Durchlässigkeit der Wertprodukte auf, sodass sich die Wertprodukte bezogen auf den Katalysator im Permeat 14 anreichern. Das Permeat 14 wird weiter zur Aufbereitung geführt (nicht dargestellt); das katalysatorreiche Retentat 15 wird mit frischem, flüssigen Edukt 16 vermischt über die Strahldüse 4 in den Reaktor 1 zurückgeführt.

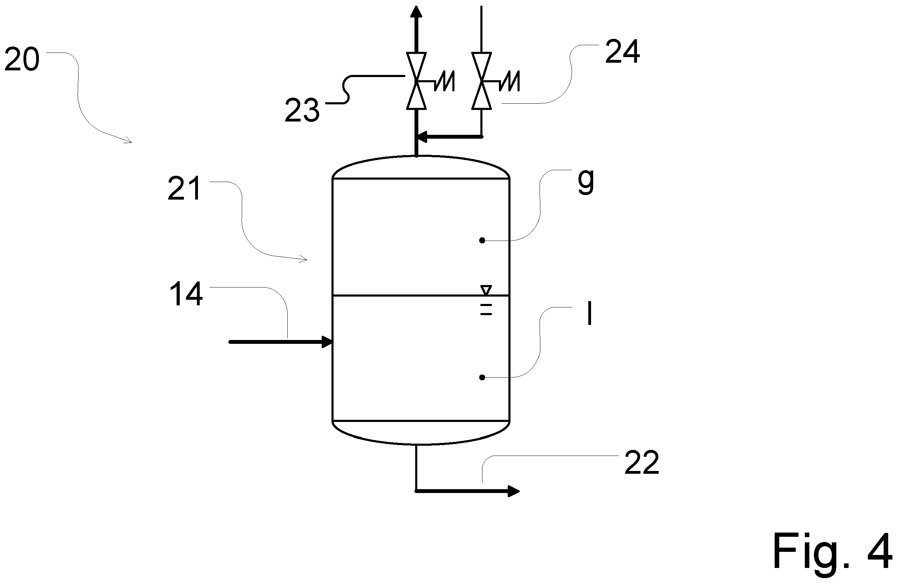

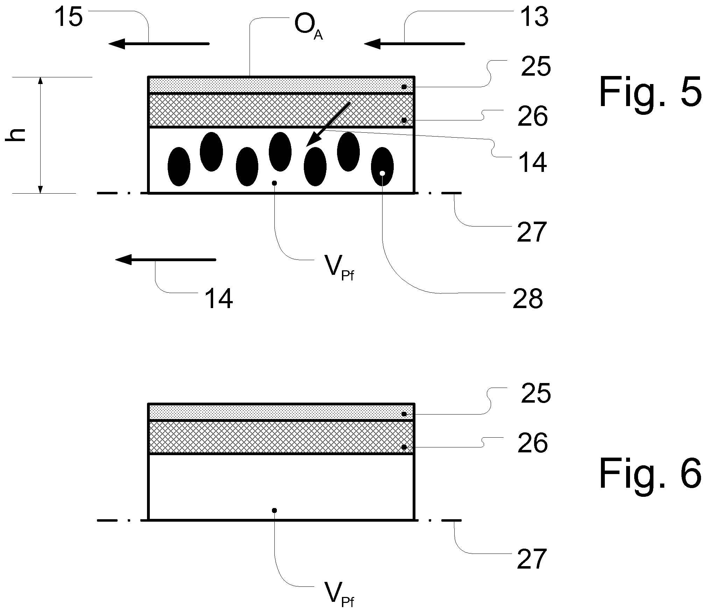

Um Schäden an der Membran und Desaktivierung des Katalysators zu verhindern, ist unkontrolliertes Entgasen von gelösten gasförmigen Reaktionsteilnehmern an der Membran 12 zu vermeiden und ein Mindest-CO-Partialdruck im Permeat 14 zu gewährleisten. Dies wird durch ein eigens vorgesehenes Entgasungsorgan (siehe unten zu Figur 4) und/oder durch die geeignete Bemessung des freien Volumens der Membran (siehe unten zu Figuren 5 bis 10) erreicht. Zusätzlich hilft die Abschirmung des Abzugs 8 mittels einer Prallplatte 7, Gasblasen im Feed 13 zur Membrantrennstufe 12 weitestgehend zu vermeiden.

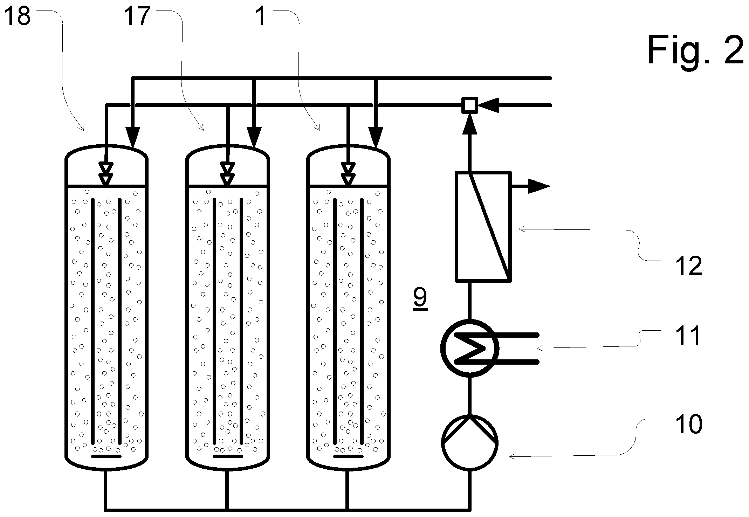

Figur 2 zeigt eine zweite Ausführungsform der erfindungsgemäßen Vorrichtung. Sie umfasst drei parallel geschaltete Strahlschlaufenreaktoren 1, 17, 18, welche jeder für sich wie der beschriebene erste Strahlschlaufenreaktor 1 aufgebaut ist. Alle drei Strahlschlaufenreaktoren 1, 17, 18 teilen sich den gemeinsamen äußeren Flüssigkeitsumlauf 9, in welchem gemeinsam Pumpe 10, Wärmetauscher 11 und Membrantrennstufe 12 genutzt wird. Vorteil der Parallelisierung ist eine bessere Anpassbarkeit der Anlage an wechselnde Nachfrage: Bei Grundlast sollten zwei Strahlschlaufenreaktoren 1, 17 laufen, bei großer Nachfrage kann der Dritte 18 hinzugeschaltet werden, bei geringer Nachfrage wird auch der Zweite 17 ausgeschaltet, sodass die Anlage nur mit einem Reaktor 1 arbeitet. Die Reaktoren 1, 17 ,18 müssen dementsprechend mit geeigneten Abschaltorganen versehen sein (nicht dargestellt). Durch die schaltbare Parallelisierung ist eine bessere Anlagenausnutzung möglich. Darüber hinaus kann jeder einzelne (zugeschaltete) Reaktor stets im strömungstechnisch optimalen Betriebszustand betrieben werden. Teillastbetrieb wird weitestgehend vermieden. Durch Veränderung der Drehzahl wird die Pumpe an die jeweiligen Volumenströme angepasst. Die Anpassung der gesamten aktiven Oberfläche der Membrantrennstufe ist ebenfalls durch geeignete Parallelschaltung von mehreren Membranen möglich.

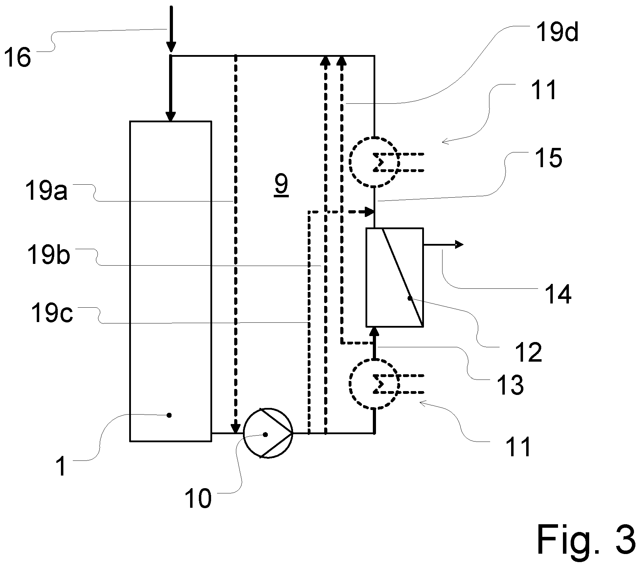

Figur 3 zeigt verschiedene Weiterbildungen der Erfindung, welche jeweils eine Bypass-Leitung 19a, 19b, 19c, 19d aufweisen.

Wie vorstehend beschrieben, umfasst die Anlage nach dieser Ausführungsform auch einen Strahlschlaufenreaktor 1, in dessen äußeren Flüssigkeitsumlauf 9 eine Pumpe 10 und eine Membrantrennstufe 12 zur Abfuhr des Produkts mit dem Permeat 14 angeordnet ist. Edukt 16 wird in den Reaktor 1 eingedüst. Ein optionaler Wärmetauscher 11 kann im Feed 14 oder im Retentat 15 der Membrantrennstufe 15 angeordnet sein. Die Aufrechterhaltung des Flüssigkeitsumlaufes 9 setzt voraus, dass der Massendurchsatz durch den Reaktor 1 genauso groß ist wie über die Membran (Edukt 16 und Produkt im Permeat 14 gleichen sich aus). Da die Verweilzeit im Reaktor aber einen anderen Massenstrom erfordern kann, als die ideale Membranüberströmung gebietet, ist je nach Prozess eine Bypassleitung 19 erforderlich, welche jeweils parallel zum "langsameren" Anlagenorgan verläuft. So kann Bypass 19a parallel zu dem langsameren Reaktor 1 verlaufen; für den Fall, dass der Reaktor schneller ist, ist der Bypass 19b parallel zur Membrantrennstufe 12 anzuordnen. Sofern der Wärmetauscher 11 den limitierenden Faktor darstellt, ist bei Wärmetauscher im Feed Bypass 19c bzw. bei Wärmetauscher im Retentat Bypass 19d vorzusehen. Durch den Bypass fließt jeweils ein entsprechender Teilstrom an dem jeweiligen Anlagenorgan vorbei. Der andere Teil fließt selbstverständlich weiter durch das Organ. Der Bypass stellt mithin keine vollständige Überbrückung dar sondern eröffnet lediglich einen hydraulisch günstigen Alternativweg.

Figur 4 zeigt eine optionale Entgasung 20, die im Permeatstrom 14 der Membrantrennstufe angeordnet werden kann, um eine unkontrollierte Entgasung an der Membran zu verhindern. Die Entgasung umfasst einen Druckbehälter 21, in welchem sich eine flüssige Phase l und eine gasförmige Phase g voneinander trennen. Die produkthaltige flüssige Phase l wird über einen Produktabzug 22 abgezogen. Der Druck der gasförmigen Phase wird mittels Drosseln 23, 24 geregelt. Ist der Druck zu groß, wird über Drossel 23 Gas abgezogen. Sinkt der Druck im Behälter, was zu einer Entgasung an der Membran führen kann, wird der Druckbehälter 21 über Drossel 24 von außen mit Gas beaufschlagt. Über die Entgasung 20 kann auch der Transmembrandruck der Membrantrennstufe 12 eingestellt werden.

Da handelsübliche Membranmodule nicht in der Lage sind, Gasanteile im Flüssigkeitsstrom dauerhaft schadlos durchzusetzen, wird erfindungsgemäß vorgeschlagen, das permeatseitig freie Volumen mit mindestens 0.3 $dm^3$ pro $m^2$ Membranfläche zu dimensionieren. Das freie permeatseitige Volumen wird anhand der Figuren 5 bis 10 erläutert.

Figur 5 zeigt den schematischen Aufbau einer ebenen Membrantrennstufe. Die Membran umfasst eine trennaktive Schicht 25 und eine stromabwärts mit dem Permeat angeordnete poröse Stützschicht 26. Die Trennung geschieht an der trennaktiven Schicht 25, die Stützschicht 26 stabilisiert die trennaktive Schicht 25 mechanisch. Das Membranmodul ist entweder symmetrisch aufgebaut (Symmetrielinie 27) oder unsymmetrisch. In diesem Fall bezeichnet die Linie 27 die gegenüberliegende Wand des Membranmoduls. Weiter stromabwärts mit dem Permeat kann ein Permeatplatzhalter 28 (auch Permeatspacer genannt) vorgesehen sein, beispielsweise eine grob poröse Struktur oder ein Gitter oder ein Geflecht.

Der Feed 13 strömt entlang der trennaktiven Schicht 25 wird um dessen permeierenden Bestandteile abgereichert und verlässt als Retentat 15 die Membran. Das Permeat 14 durchdringt die trennaktive Schicht 26 und verlässt die Membrantrennstufe. Das permeatseitige Volumen $V_P$ versteht sich als das permeatseitig senkrecht zur aktiven Membranoberfläche $O_A$ (Oberfläche der trennaktiven Schicht 25) anschließende Volumen. Dieses erstreckt sich entlang der Höhe h bis zur Symmetrielinie bzw. Wand 27 der Membrantrennstufe. Damit gilt für ebene Membransysteme oder auch in Näherung für Flachkanalsysteme wie dem Spiralwickelelement:

$$V_P = O_A * h.$$

Das freie permeatseitige Volumen $V_{Pf}$ ist der Anteil des permeatseitigen Volumens $V_P$, welches nicht von der Materie der trennaktiven Schicht 25, der Stützschicht 26 und dem Permeatplatzhalter 28 gefüllt ist. Dieser Raum wird im Betrieb von Permeat eingenommen. Er lässt sich dementsprechend durch Einfüllen einer Prüfflüssigkeit volumetrisch messen (so genanntes "Auslitern").

Das freie permeatseitige Volumen $V_{Pf}$ wird durch Dimensionierung des Permeatplatzhalters 28 eingestellt, da die Porosität der trennaktiven Schicht 25 durch die Trennaufgabe und die der Stützschicht 26 durch die mechanische Belastung determiniert ist.

Das permeatseitige freie Volumen $V_{Pf}$ ist erfindungsgemäß so einzustellen, dass gilt:

$$V_{Pf}\ [dm^3] \geq 0.3^*\ O_A\ [m^2]$$

Durch den in Figur 6 gezeigten Verzicht auf einen Permeatplatzhalter wird das permeatseitige freie Volumen $V_{Pf}$ erhöht, sofern die übrigen Abmessungen der Membran beibehalten werden.

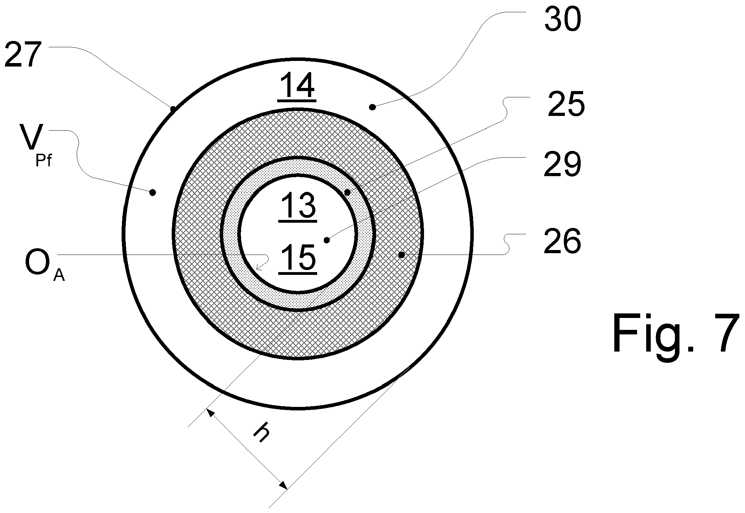

In den Figuren 5 und 6 wurde die Membran von außen nach innen durchströmt. Es gibt auch Membranmodule, bei denen die Membranen von innen nach außen durchströmt werden. So skizziert Figur 7 ein rohrförmiges Membranmodul, bei welcher Feed 13 und Pemeat 15 axial durch einen zylindrischen inneren Kanal 29 strömen, welcher von der trennaktiven Schicht 25 umgeben ist. Auf diese folgt radial nach außen die Stützschicht 26. Das Permeat 14 fließt durch einen ringförmigen äußeren Kanal 30 aus dem Membranmodul ab, welcher die Stützschicht 26 umgibt und selbst von der Wand 27 des Membranmoduls nach außen abgeschlossen ist. Das permeatseitige Volumen $V_P$ bereichnet sich damit aus dem Produkt der aktiven Membranoberfläche $O_A$ und der Höhe h.

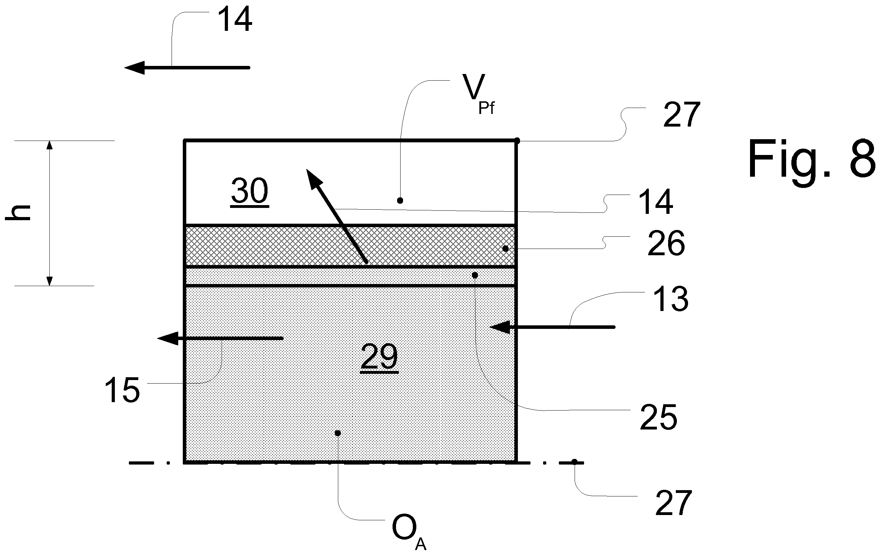

Figur 8 zeigt einen Längsschnitt durch das rohrförmige Membranmodul aus Figur 7.

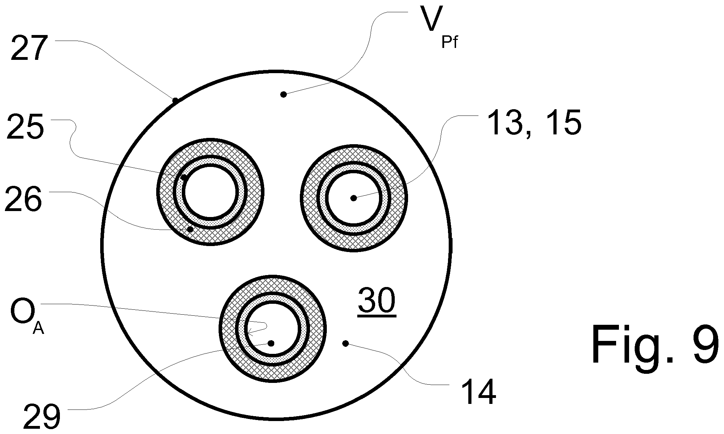

Figur 9 zeigt eine Variante eines rohrförmigen Membranmoduls, bei welcher eine Vielzahl von zylindrischen inneren Kanälen 29 innerhalb der Wandung 27 eines Rohrs gebündelt sind. Jeder innere Kanal 29 ist von einer zylindrischen trennaktiven Schicht 25 und einer zylindrischen Stützschicht 26 umgeben. Bei der Berechnung des permeatseitigen Volumens ist mithin die Packungsdichte der Kanäle 29 zu berücksichtigen. Für die aktive Membranoberfläche $O_A$ ist die Summe der von der trennaktiven Schicht 25 gebildeten Mantelflächen der inneren Kanäle 29 anzusetzen. Durch wahlweises Abschalten der einzelnen inneren Kanäle 29 durch geeignete Abschaltorgane lässt sich eine schaltbare Parallelisierung der Membranen realisieren. Damit ist eine flexible Anpassung der gesamten aktiven Oberfläche der Membrantrennstufe an die Bedarfslage möglich.

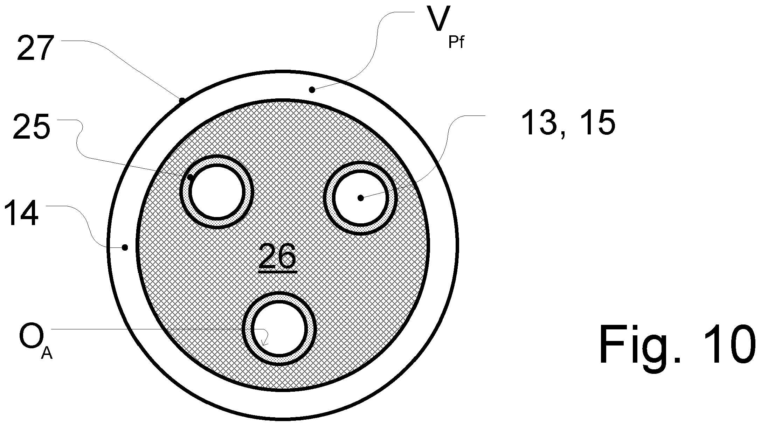

Figur 10 zeigt eine weitere Variante eines rohrförmigen Membranmoduls, bei welcher eine Vielzahl von inneren Kanälen 29 innerhalb der Wandung 27 eines Rohrs gebündelt sind, wobei sich die inneren Kanäle 29 jedoch eine gemeinsame Stützstruktur 26 teilen.

Mit der erfindungsgemäßen Kombination aus einem Strahlschlaufenreaktor mit einer Membrantrennstufe lassen sich gas/flüssig-Reaktionen, insbesondere Hydroformylierungen, wirtschaftlicher durchführen als mit traditionell in der Industrie eingesetzten Reaktoren.

**Beispiele**

1. Membranvortestung

Für die Vorauswahl geeigneter Membranen wurden Versuche zur Permeatflussbestimmung und Rückhaltmessung durchgeführt. In einem 5L Vorlagebehälter wurde ein Reaktionsgemisch einer Hydroformylierung von 1-Penten vorgelegt. Die Zusammensetzung des Reaktionsgemisches ist in Tabelle X angegeben. Als Katalysator-Ligandsystem sind 10 mg/kg Rhodium und 1170 mg/kg Alkanox P-24 (CAS-Nr. 26741-53-7) enthalten.

Tabelle X:

| 1-Penten | 2-Methylpentanal | Hexanal | Rest |
|---|---|---|---|
| [Gew-%] | [Gew-%] | [Gew-%] | [Gew-%] |
| 6,996 | 47,960 | 43,932 | 1,112 |
| 6,894 | 47,774 | 43,585 | 1,747 |

Das Reaktionsgemisch wurde bei 25°C, einer transmembranen Druckdifferenz von 4 bar und einer Überströmung von 200 l/h über die getesteten Membranen geführt.

Getestet wurden $TiO_2$ Monokanalmembran des Typs Innopor® nano mit einer mittleren Porengröße von 0,9nm, einer Trenngrenze von 450 Da und einer offenen Porosität von 0,3 bis 0,4 der Firma Innopor GmbH. Bei einer Kanallänge von 500mm und einem Innendurchmesser von 7 mm ergibt sich eine aktive Membranfläche von ca. 100 cm2.

Die Ergebnisse der Permeabilitätsmessungen zeigt Tabelle Y. Es ergeben sich Permeabilitäten in zwei unterschiedlichen Größenordnungen. Vier von acht getesteten Membranen liegen im Bereich von 0,8 bis 2,9 kg/($m^2$h bar).

Die anderen vier Membranen liegen im Bereich von 18 bis 37 kg/(m$^2$h bar).

Tabelle Y:

| Membran Nummer | Permeabilität [kg/(m$^2$h bar)] |
|---|---|
| 1 | 0,8 |
| 2 | 19 |
| 3 | 37 |
| 4 | 18 |
| 5 | 29 |
| 6 | 1,6 |
| 7 | 1,5 |
| 8 | 2,9 |

In einem weiteren Test zur Rückhaltbestimmung wurde in die Vorlage Isopropanol gefüllt. Als Marker für die Rückhaltbestimmung wurde Bengalrosa (Cas-Nr. 11121-48-5) mit einer molaren Masse von 974 g/mol eingesetzt. Für die Membranen mit Permeabilitäten Bereich von 0,8 bis 2,9 kg/(m$^2$h bar) ergaben sich Rückhalte von 93 bis 97%. Für die Membranen mit Permeabilitäten im Bereich von 18 bis 37 kg/(m$^2$h bar) ergaben sich Rückhalte von 55 bis 61 %. Da die Membranen laut Herstellerangabe eine Trenngrenze von 450 g/mol besitzen sollen, wurden die Membranen mit der hohen Permeabilität und dem niedrigen Rückhalt für Bengalrosa als defekt aussortiert.

2. Erfindungsgemäßes Beispiel (0,9 nm)

In einer wie in Fig. 1 dargestellten Versuchsanlage mit einem Strahlschlaufenreaktor (1) wurden Hydroformylierungsreaktionen von 1-Penten (16) mit Synthesegas (5) zu den entsprechenden Aldehydisomeren durchgeführt. Im von einer Peripheralradpumpe (10) getriebenen Flüssigkeitsumlauf (9) erfolgte eine Abtrennung und Rückführung des Katalysator-Ligandsystems mittels einer Membrantrennstufe (12) zur kontinuierlichen Wiedereinsetzung des Katalysator-Ligandsystems in der Hydroformylierungsreaktion im Strahlschlaufenreaktor (1).

Für die Reaktion wurden dem Reaktor 1-Penten (16) unter Sauerstoffausschluss kontinuierlich entsprechend der Reaktionsproduktabfuhr über das Permeat der Membrantrennstufe zugeführt. Der Katalysatorpräkursor war Rhodiumacetylacetonatodicarbonyl (CAS-Nr. 14847-82-9). Als Ligand wurde Alkanox P-24 (CAS-Nr. 26741-53-7) eingesetzt. Die Rhodiumkonzentration und die Ligandkonzentration im Schlaufenreaktor wurde durch eine kontinuierliche Nachdosierung konstant auf 10 mg/kg bzw. 1170 mg/kg gehalten. Die Reaktion wurde unter 50 bar Synthesegasdruck (CO/H$_2$, Massenverhältnis 1:1), bei 110 °C durchgeführt.

Das Reaktionsprodukt wurde kontinuierlich über eine als einstufige Nanofiltrationsmembran ausgeführte Membrantrennstufe (12) geführt. Der benötigte transmembrane Druck wird durch den Reaktordruck und einem geregelten permeatseitigen (14) Druck aufgebaut. Die gewünschte Überströmung von 500 kg/h über die Hochdruckseite der Membran wird über die Peripheralradpumpe eingestellt.

Im Membranmodul der Membrantrennstufe (12) war Membran Nummer 1 aus Beispiel 1 eingebaut.

Bei einer Kanallänge von 500mm und einem Innendurchmesser von 7 mm ergibt sich eine aktive Membranfläche von ca. 100 cm$^2$. Das auf die Membranfläche bezogene permeatseitige freie Volumen ist größer als 0,5 dm$^3$/m$^2$. Die Membran wurde mit 4,4 m/s überströmt. Die Temperatur im Membrantrennschritt betrug 102°C. Zur Stabilisierung des Katalysator-Ligandkomplexes wurde auf der Permeatseite ein Synthesegasdruck (CO/H2, Massenverhältnis 1:1) von 10 bar mit einer Vorrichtung gemäß Abbildung 4 gehalten, wodurch bei einem retentatseitigen Druck von 50 bar ein transmembranen Druck von 40 bar eingestellt wurde. Der niedrige Druck der Permeatseite führt zu einem Ausgasen von Synthesegas. Der zweiphasige Permeatstrom (14) wird eine Entgasungseinrichtung (Fig. 4) geführt.

In der Membrantrennstufe wurde dem System über die Membran Permeat (14), das vorwiegend aus Reaktionsprodukt besteht, entnommen. Der Katalysator sowie der Ligand Alkanox wurde von der Membran weitestgehend zurückgehalten und reichersich im Retentat (15) an. Das Retentat (15) wurde kontinuierlich zurück in den Strahlschlaufenreaktor (1) zugeführt.

Die Prozesskette wurde an Hand von Mess- und Analysendaten, die durch gaschromatographische Analyse, HPLC-Analyse, Atomabsorptionsspektroskopie und optische Emissionsspektrometrie mit induktiv gekoppeltem Hochfrequenzplasma erhalten wurden, ausgewertet. Die Reaktion wurde hinsichtlich des Umsatzes an 1-Penten sowie der Ausbeute und der Selektivität an Aldehyd untersucht. Die Membrantrennstufe (12) wurde hinsichtlich Permeatfluss und Rückhalt für Rhodium und Ligand untersucht. Der Umsatz von 1-Penten lag bei 95% und die Aldehydselektivität lag bei 98%.

Die TiO$_2$ Monokanalmembran des Typs Innopor® nano zeigte bei spezifischen Permeatflussleistungen zwischen 53 und 57 kg/m$^2$h gegenüber dem Rhodium einen Membranrückhalt zwischen 88 und 92 %. Der Rückhalt des Liganden lag bei 83%.

Das Beispiel zeigt, dass mit der gewählten Membrantrennstufe im Flüssigkeitsumlauf des Schlaufenreaktors der Homogenkatalysator quantitativ zurückgehalten und dem Schlaufenreaktor zurückgeführt werden konnte. Das ausreichend hohe spezifische freie Permeatvolumen der Membrantrennstufe ermöglichte eine hohen Rückhalt des Katalysatorsystems und einen uneingeschränkt guten Permeatfluss trotz der permeatseitigen Entgasung. Zudem wurde der Katalysator unter den gewählten Bedingungen in aktiver Form mit einer Nanofiltration zurückgehalten.

### 3. Erfindungsgemäßes Beispiel (3nm)

In einer wie in Fig. 1 dargestellten Versuchsanlage mit einem Strahlschlaufenreaktor (1) wurden Hydroformylierungsreaktionen von 1-Penten (16) mit Synthesegas (5) zu den entsprechenden Aldehydisomeren durchgeführt. Im von einer Peripheralradpumpe (10) getriebenen Flüssigkeitsumlauf (9) erfolgte eine Abtrennung und Rückführung des Katalysator-Ligandsystems mittels einer Membrantrennstufe (12) zur kontinuierlichen Wiedereinsetzung des Katalysator-Ligandsystems in der Hydroformylierungsreaktion im Strahlschlaufenreaktor (1).

Für die Reaktion wurden dem Reaktor 1-Penten (16) unter Sauerstoffausschluss kontinuierlich entsprechend der Reaktionsproduktabfuhr über das Permeat der Membrantrennstufe zugeführt. Der Katalysatorpräkursor war Rhodiumacetylacetonatodicarbonyl (CAS-Nr. 14847-82-9). Als Ligand wurde Alkanox P-24 (CAS-Nr. 26741-53-7) eingesetzt. Die Rhodiumkonzentration und die Ligandkonzentration im Schlaufenreaktor wurden durch eine kontinuierliche Nachdosierung konstant auf 10 mg/kg bzw. 1170 mg/kg gehalten. Die Reaktion wurde unter 50 bar Synthesegasdruck (CO/H$_2$, Massenverhältnis 1:1), bei 110 °C durchgeführt.

Das Reaktionsprodukt wurde kontinuierlich über eine als einstufige Nanofiltrationsmembran ausgeführte Membrantrennstufe (12) geführt. Der benötigte transmembrane Druck wird durch den Reaktordruck und einem geregelten permeatseitigen (14) Druck aufbaut. Die gewünschte Überströmung von 500 kg/h über die Hochdruckseite der Membran wird über die Peripheralradpumpe eingestellt.

Im Membranmodul der Membrantrennstufe (12) war ein Prototyp einer durch Silanisierung hydrophobierten Membran als Monokanalrohr vom Fraunhofer-Institut für Keramische Technologien und Systeme IKTS verbaut. Der Träger bestand aus Al2O3 mit einer mittleren Porengröße von 3 $\mu$m und einer hydrophobierten Membranschicht basierend auf einer ZrO2 Schicht mit einem mittleren Porendurchmesser von 3 nm.

Bei einer Kanallänge von 500mm und einem Innendurchmesser von 7 mm ergibt sich eine aktive Membranfläche von ca. 100 cm$^2$. Das auf die Membranfläche bezogene permeatseitige freie Volumen ist größer als 0,5 dm$^3$/m$^2$. Die Membran wurde mit 4,4 m/s überströmt. Die Temperatur im Membrantrennschritt betrug 101°C. Zur Stabilisierung des Katalysator-Ligandkomplexes wurde auf der Permeatseite ein Synthesegasdruck (CO/H$_2$, Massenverhältnis 1:1) von 10 bar mit einer Vorrichtung gemäß Abbildung 4 gehalten, wodurch bei einem retentatseitigen Druck von 50 bar ein transmembranen Druck von 40 bar eingestellt wurde. Der niedrige Druck der Permeatseite führt zu einem Ausgasen von Synthesegas. Der zweiphasige Permeatstrom (14) wird eine Entgasungseinrichtung (Fig. 4) zugeführt.

In der Membrantrennstufe wurde dem System über die Membran Permeat (14), das vorwiegend aus Reaktionsprodukt besteht, entnommen. Der Katalysator sowie der Ligand Alkanox wurde von der Membran weitestgehend zurückgehalten und reichert sich im Retentat (15) an. Das Retentat (15) wurde kontinuierlich zurück in den Strahlschlaufenreaktor (1) geführt.

Die Prozesskette wurde an Hand von Mess- und Analysendaten, die durch gaschromatographische Analyse, HPLC-Analyse, Atomabsorptionsspektroskopie und Optische Emissionsspektrometrie mit induktiv gekoppeltem Hochfrequenzplasma erhalten wurden, ausgewertet. Die Reaktion wurde hinsichtlich des Umsatzes an 1-Penten sowie der Ausbeute und der Selektivität an Aldehyd untersucht. Die Membrantrennstufe (12) wurde hinsichtlich Permeatfluss und Rückhalt für Rhodium und Ligand untersucht. Der Umsatz von 1-Penten lag bei 94% und die Aldehydselektivität lag bei 98%.

Die Prototypmembran zeigte bei spezifischen Permeatflussleistungen zwischen 96 und 98 kg/m$^2$h gegenüber dem Rhodium einen Membranrückhalt zwischen 73 und 74 %. Der Rückhalt des Liganden lag bei 64%.

Auch Beispiel 2 zeigt, dass mit der gewählten Membrantrennstufe im Flüssigkeitsumlauf des Schlaufenreaktors der Homogenkatalysator quantitativ zurückgehalten und dem Schlaufenreaktor zurückgeführt werden konnte. Das ausreichend hohe spezifische freie Permeatvolumen der Membrantrennstufe ermöglichte einen hohen Rückhalt des Katalysatorsystems und einen uneingeschränkt guten Permeatfluss trotz der permeatseitigen Entgasung. Zudem wurde der Katalysator unter den gewählten Bedingungen in aktiver Form mit einer Nanofiltration zurückgehalten.

4. Beispiel

In diesem Beispiel soll der Einfluss der Gasbelastung bei möglichst konstanten Bedingungen in der Membrantrennstufe bzgl. Retentatdruck, Permeatdruck und Temperatur gezeigt werden. Hierzu wurde der Versuchsaufbau aus Beispiel 2 und Beispiel 3 gemäß Figur 1 um eine druckerzeugende Pumpe im Flüssigkeitsumlauf (9) nach dem Schlaufenreaktor (1) und vor der Bypass-Leitung (19a, Fig. 3) erweitert. Dies ist notwendig, da bei den Versuchen der Feeddruck der Membran teilweise oberhalb des Synthesegasdrucks im Reaktor liegen soll. Die Peripheralradpumpe (10) erzeugt weiterhin die Überströmung der Membrantrennstufe.

Im Strahlschlaufenreaktor (1) wurde Hydroformylierungsprodukt von 1-Penten (16) gemäß der Zusammensetzung von Tabelle X aus Beispiel 1 vorgelegt. Im von der Peripheralradpumpe (10) getriebenen Flüssigkeitsumlauf (9) erfolgte eine Abtrennung und Rückführung des Katalysator-Ligandsystems mittels einer Membrantrennstufe (12) zum Katalysator-Ligandsystems in den Strahlschlaufenreaktor (1).

Der Synthesegasdruck ($CO/H_2$, Massenverhältnis 1:1) wurde im Strahlschlaufenreaktor (1) von 10, 20, 30, 40, 50 bar variiert.

Das mit Synthesegas beaufschlagte Reaktionsprodukt wurde kontinuierlich über eine als einstufige Nanofiltrationsmembran ausgeführte Membrantrennstufe (12) geführt. Der benötigte transmembrane Druck wird durch die zusätzlich Pumpe im Flüssigkeitsumlauf auf 40 bar gehalten. Die gewünschte Überströmung von 500 kg/h über die Hochdruckseite der Membran wird über die Peripheralradpumpe eingestellt.

Im Membranmodul der Membrantrennstufe (12) war Membran Nummer 1 aus Beispiel 1 eingebaut. Bei einer Kanallänge von 500mm und einem Innendurchmesser von 7 mm ergibt sich eine aktive Membranfläche von ca. 100 $cm^2$. Das auf die Membranfläche bezogene permeatseitige freie Volumen ist größer als 0,5 $dm^3/m^2$. Die Membran wurde mit 4,4 m/s überströmt. Die Temperatur im Membrantrennschritt betrug 60°C. Zur Stabilisierung des Katalysator-Ligandkomplexes wurde auf der Permeatseite ein Synthesegasdruck ($CO/H_2$, Massenverhältnis 1:1) von 10 bar mit einer Vorrichtung gemäß Abbildung 4 gehalten, wodurch bei einem retentatseitigen Druck von 50 bar ein transmembranen Druck von 40 bar eingestellt wurde. Der niedrige Druck der Permeatseite führt je nach Höhe des Synthesegasdrucks im Schlaufenreaktor zu einer entsprechenden Menge an ausgasendem Synthesegas auf der Permeatseite. Der zweiphasige Permeatstrom (14) wird eine Entgasungseinrichtung (Fig. 4) zugeführt.

In der Membrantrennstufe wurde dem System über die Membran Permeat (14), das vorwiegend aus Reaktionsprodukt besteht, entnommen. Der Rhodium-Komplex sowie der freie Ligand wurden von der Membran weitestgehend zurückgehalten und reichern sich im Retentat (15) an. Das Retentat (15) wurde kontinuierlich zurück in den Strahlschlaufenreaktor (1) geführt.

Die Prozesskette wurde an Hand von Mess- und Analysendaten, die durch gaschromatographische Analyse, HPLC-Analyse, Atomabsorptionsspektroskopie und optische Emissionsspektrometrie mit induktiv gekoppeltem Hochfrequenzplasma erhalten wurden, ausgewertet. Die Membrantrennstufe (12) wurde hinsichtlich Permeatfluss und Rückhalt für Rhodium und Ligand untersucht. Die Ergebnisse des Beispiels fasst Tabelle Z1 zusammen:

Tabelle Z1:

| Synthesegas | TMP | Temperatur | Permeatfluss | Rh-Rückhalt |
|---|---|---|---|---|
| [bar] | [bar] | [°C] | [kg/($m^2$h)] | [%] |
| 10 | 40 | 60 | 28,2 | 91,1 |
| 20 | 40 | 60 | 30,1 | 89,8 |
| 30 | 40 | 60 | 29,4 | 90,5 |
| 40 | 40 | 60 | 28,4 | 91,3 |
| 50 | 40 | 60 | 29,7 | 90,0 |

Dieses Beispiel zeigt, dass der Synthesegasdruck im Reaktor und damit die Menge an gelöstem Synthesegas bei einem auf die Membranfläche bezogenen permeatseitigen freien Volumen von mehr als 0,5 $dm^3/m^2$ keinen Einfluss auf den Permeatfluss besitzt.

5. Gegenbeispiel im Membranwickel

In diesem Beispiel soll der Einfluss der Gasbelastung bei möglichst konstanten Bedingungen in der Membrantrennstufe bzgl. Retentatdruck, Permeatdruck und Temperatur gezeigt werden. Hierzu wurde der Versuchsaufbau aus Beispiel 2 und Beispiel 3 gemäß Figur 1 um eine druckerzeugende Pumpe im Flüssigkeitsumlauf (9) nach dem Schlaufenreaktor (1) und vor der Bypass-Leitung (19a, Fig. 3) erweitert. Dies ist notwendig, da bei den Versuchen der Feeddruck der Membran teilweise oberhalb des Synthesegasdrucks im Reaktor liegen soll. Die Peripheralradpumpe (10) erzeugt weiterhin die Überströmung der Membrantrennstufe.

Im Strahlschlaufenreaktor (1) wurde Hydroformylierungsprodukt von 1-Penten (16) gemäß der Zusammensetzung von Tabelle X aus Beispiel 1 vorgelegt. Im von der Peripheralradpumpe (10) getriebenen Flüssigkeitsumlauf (9) erfolgte eine Abtrennung und Rückführung des Katalysator-Ligandsystems mittels einer Membrantrennstufe (12) zur des Katalysator-Ligandsystems in den Strahlschlaufenreaktor (1). Der Synthesegasdruck (CO/H2, Massenverhältnis 1:1) wurde im Strahlschlaufenreaktor (1) von 10, 20, 30, 40, 50 bar variiert.

Das mit Synthesegas beaufschlagte Reaktionsprodukt wurde kontinuierlich über eine als einstufige Nanofiltrationsmembran ausgeführte Membrantrennstufe (12) geführt. Der benötigte transmembrane Druck wird durch die zusätzlich Pumpe im Flüssigkeitsumlauf auf 40 bar gehalten. Die gewünschte Überströmung von 250 kg/h über die Hochdruckseite der Membran wird über die Peripheralradpumpe eingestellt.

Im Membranmodul der Membrantrennstufe (12) war ein 1,8" mal 12" Membranspiralwickel mit einer Membran vom Typ ONF2 der Firma GMT Membrantechnik GmbH verbaut. Der Feedspacer hatte eine Höhe von 31 mm und der Permeatspacer hatte eine Höhe von 10 mm. Daraus ergibt sich eine aktive Membranfläche von ca. 0,1 m$^2$. Das auf die Membranfläche bezogene permeatseitige freie Volumen ist kleiner als 0,1 dm$^3$/m$^2$. Die Temperatur im Membrantrennschritt betrug 60°C. Zur Stabilisierung des Rhodium-Ligandkomplexes wurde auf der Permeatseite ein Synthesegasdruck (CO/H2, Massenverhältnis 1:1) von 10 bar mit einer Vorrichtung gemäß Abbildung 4 gehalten, wodurch bei einem retentatseitigen Druck von 50 bar ein transmembranen Druck von 40 bar eingestellt wurde. Der niedrige Druck der Permeatseite führt je nach Höhe des Synthesegasdrucks im Schlaufenreaktor zu einer entsprechenden Menge an ausgasendem Synthesegas auf der Permeatseite. Der zweiphasige Permeatstrom (14) wird eine Entgasungseinrichtung (Fig. 4) geführt.

In der Membrantrennstufe wurde dem System über die Membran Permeat (14), das vorwiegend aus Reaktionsprodukt besteht, entnommen. Der Rhodium-Komplex sowie der freie Ligand wurden von der Membran weitestgehend zurückgehalten und reichern sich im Retentat (15) an. Das Retentat (15) wurde kontinuierlich zurück in den Strahlschlaufenreaktor (1) geführt.

Die Prozesskette wurde an Hand von Mess- und Analysendaten, die durch gaschromatographische Analyse, HPLC-Analyse, Atomabsorptionsspektroskopie und Optische Emissionsspektrometrie mit induktiv gekoppeltem Hochfrequenzplasma erhalten wurden, ausgewertet. Die Membrantrennstufe (12) wurde hinsichtlich Permeatfluss und Rückhalt für Rhodium und Ligand untersucht. Die Ergebnisse des Beispiels fasst Tabelle Z2 zusammen:

Tabelle Z2:

| Synthesegas | TMP | Temperatur | Permeatfluss | Rh-Rückhalt |
|---|---|---|---|---|
| [bar] | [bar] | [°C] | [kg/(m$^2$h)] | [%] |
| 10 | 40 | 60 | 114 | 96,4 |
| 20 | 40 | 60 | 114 | 94,8 |
| 30 | 40 | 60 | 113 | 96,3 |
| 40 | 40 | 60 | 101 | 93,3 |
| 50 | 40 | 60 | 79 | 94,0 |

Dieses Beispiel zeigt, dass der Synthesegasdruck im Reaktor und damit die Menge an gelöstem Synthesegas einen Einfluss auf den Permeatfluss besitzt. Je mehr Synthesegas auf der Permeatseite der Membran ausgasen kann desto geringer wird der Permeatfluss der Membran. Im Beispiel ist der Permeatfluss bei einem reaktionstypischen Synthesegasdruck von 50 bar um 31% geringer als bei einem Synthesegasdruck von 10 bis 20 bar im Reaktor. Ursächlich hierfür ist das zu geringe permeatseitigen Volumen von weniger als 0,1 dm$^3$/m$^2$.

6. Beispiel: Langzeittestung

Die Membran aus Beispiel 5 wurde in einem Langzeitversuch untersucht. Die Reaktionsführung entspricht den Beispielen 2 und 3. Die Reaktion wurde unter 50 bar Synthesegasdruck (CO/H2, Massenverhältnis 1:1), bei 110 °C durchgeführt.

Das Reaktionsprodukt wurde zunächst über einen Wärmetauscher (11, Fig. 3) und anschließend über eine als einstufige Nanofiltrationsmembran ausgeführte Membrantrennstufe (12) geführt. Die Temperatur im Membrantrennschritt betrug 60°C. Der benötigte transmembrane Druck wird durch den Reaktordruck und einem geregelten permeatseitigen (14) Druck aufbaut. Die gewünschte Überströmung von 250 kg/h über die Hochdruckseite der Membran wird über die Peripheralradpumpe eingestellt. Zur Stabilisierung des Rhodium-Ligandkomplexes wurde auf der Permeatseite ein Synthesegasdruck (CO/$H_2$, Massenverhältnis 1:1) von 10 bar mit einer Vorrichtung gemäß Abbildung 4 gehalten, wodurch bei einem retentatseitigen Druck von 50 bar ein transmembranen Druck von 40 bar eingestellt wurde. Der niedrige Druck der Permeatseite führt zu einem Ausgasen von Synthesegas. Der zweiphasige Permeatstrom (14) wird eine Entgasungseinrichtung (Fig. 4) geführt. Ein zweiter Wärmetauscher (11, Fig. 3) im Flüssigkeitsumlauf erwärmt den Kreislauf wieder auf Reaktionstemperatur.

In der Membrantrennstufe wurde dem System über die Membran Permeat (14), das vorwiegend aus Reaktionsprodukt besteht, entnommen. Der Rhodium-Komplex sowie der freie Ligand wurden von der Membran weitestgehend zurückgehalten und reicherten sich im Retentat (15) an. Das Retentat (15) wurde kontinuierlich zurück in den Strahlschlaufenreaktor (1) geführt.

Die Prozesskette wurde an Hand von Mess- und Analysendaten, die durch gaschromatographische Analyse, HPLC-Analyse, Atomabsorptionsspektroskopie und optische Emissionsspektrometrie mit induktiv gekoppeltem Hochfrequenzplasma erhalten wurden, ausgewertet. Die Reaktion wurde hinsichtlich des Umsatzes an 1-Penten sowie der Ausbeute und der Selektivität an Aldehyd untersucht. Die Membrantrennstufe (12) wurde hinsichtlich Permeatfluss und Rückhalt für Rhodium und Ligand untersucht. Der Umsatz von 1-Penten lag bei 93% und die Aldehydselektivität lag bei 98%.

Der ONF2 Membranwickel zeigte bei spezifischen Permeatflussleistungen zwischen 73 und 77 kg/m$^2$h. Der Rhodiumrückhalt nahm innerhalb von 9 Wochen nur unwesentlich von 93 bis 94 % auf 90 % ab. In der 10. Woche wurde jedoch nur noch ein Rückhalt von 34 % gemessen. Eine solche Membranschädigung tritt im Falle einer einphasigen Strömung im Permeatraum eines solchen Membranwickels nicht auf.

**Bezugszeichenliste**

| | |
|---|---|
| 1 | Strahlschlaufenreaktor |
| 2 | Reaktionsraum |
| 3 | Flüssigkeitsspiegel |
| 4 | Strahldüse |
| 5 | Gaszufuhr |
| 6 | Leitrohr |
| 7 | Prallplatte |
| 8 | Abzug |
| 9 | äußerer Flüssigkeitsumlauf |
| 10 | Pumpe |
| 11 | Wärmetauscher |
| 12 | Membrantrennstufe |
| 13 | Feed |
| 14 | Permeat |
| 15 | Retentat |
| 16 | Edukt |
| 17 | zweiter Strahlschlaufenreaktor |
| 18 | dritter Strahlschlaufenreaktor |
| 19 | Bypass-Leitung |
| 20 | Entgasung |
| 21 | Druckbehälter |
| 22 | Produktabzug |
| 23 | Drossel Gasabzug |
| 24 | Drossel Gaszuschlag |
| g | gasförmige Phase im Druckbehälter |
| l | flüssige Phase im Druckbehälter |
| 25 | trennaktive Schicht |
| 26 | Stützschicht / Stützstruktur |
| 27 | Symmetrielinie / Wand |
| 28 | Permeatplatzhalter |
| $V_P$ | permeatseitiges Volumen |
| $V_{Pf}$ | permeatseitiges freies Volumen |

$O_A$   aktive Membranoberfläche
h   Höhe
29   innerer Kanal
30   äußerer Kanal

**Patentansprüche**

1. Vorrichtung für die kontinuierliche, homogenkatalytische Umsetzung einer Flüssigkeit mit einem Gas und gegebenenfalls einem weiteren Fluid, wobei die Vorrichtung mindestens einen Reaktor mit einem von einer Pumpe getriebenen, äußeren Flüssigkeitsumlauf umfasst, und wobei die Vorrichtung mindestens eine den Homogenkatalysator bevorzugt zurückhaltende Membrantrennstufe mit einem molecular weight cut-off (MWCO) von 150 g/mol bis kleiner 1000 g/mol aufweist,
**dadurch gekennzeichnet,**
**dass** es sich bei dem Reaktor um einen Strahlschlaufenreaktor handelt, dass Pumpe und Membrantrennstufe in demselben äußeren Flüssigkeitsumlauf angeordnet sind,
und **dass** in der Membrantrennstufe ein permeatseitig freies Volumen vorgesehen ist, welches pro m$^2$ Membranfläche mindestens 0.3 dm$^3$ beträgt.

2. Vorrichtung nach Anspruch 1,
**dadurch gekennzeichnet,**
**dass** die Pumpe baulich zum dauerhaften Pumpen von Fluiden eingerichtet ist, welche flüssige und gasförmige Phasen enthalten.

3. Vorrichtung nach Anspruch 2,
**dadurch gekennzeichnet,**
**dass** es sich bei der Pumpe um eine Peripheralradpumpe handelt.

4. Vorrichtung nach einem der vorhergehenden Ansprüche, **gekennzeichnet durch** eine permeatseitig stromabwärts hinter der Membrantrennstufe angeordnete Gasabfuhr.

5. Vorrichtung nach einem der vorhergehenden Ansprüche,
**dadurch gekennzeichnet,**
**dass** im äußeren Flüssigkeitsumlauf, insbesondere stromaufwärts vor der Membrantrennstufe, ein Wärmetauscher zum Heizen oder Kühlen des Feeds oder des Permeats der Membrantrennstufe angeordnet ist.

6. Vorrichtung nach einem der vorhergehenden Ansprüche,
**dadurch gekennzeichnet,**
**dass** sich in dem Strahlschlaufenreaktor ein rohrförmiger Reaktionsraum erstreckt, in welchen eine Strahldüse zum Eindüsen der Flüssigkeit in den Reaktionsraum sowie ein Saugrohr zum Ansaugen des Gases gemeinsam münden, und an welchem ein von einer Prallplatte abgeschirmter Abzug für den äußeren Flüssigkeitsumlauf vorgesehen ist.

7. Vorrichtung nach einem der vorhergehenden Ansprüche, **gekennzeichnet durch** mindestens eine Bypassleitung, welche im äußeren Flüssigkeitsumlauf parallel zum Strahlschlaufenreaktor oder zur Membrantrennstufe angeordnet ist.

8. Vorrichtung nach einem der vorhergehenden Ansprüche,
**dadurch gekennzeichnet,**
**dass** eine Vielzahl von parallel schaltbaren Strahlschlaufenreaktoren mit einem gemeinsamen äußeren Flüssigkeitsumlauf vorgesehen ist, wobei die Membrantrennstufe innerhalb des gemeinsamen äußeren Flüssigkeitsumlauf angeordnet ist.

9. Vorrichtung nach Anspruch 8,
**dadurch gekennzeichnet,**
**dass** die Membrantrennstufe eine Vielzahl von parallel schaltbaren Membranen umfasst, dergestalt, dass die gesamte aktive Membranoberfläche der Membrantrennstufe durch Zu- und Abschalten der Membranen einstellbar ist.

10. Verfahren zur homogenkatalytischen Umsetzung einer Flüssigkeit mit einem Gas und gegebenenfalls einem weiteren Fluid,

**dadurch gekennzeichnet,**

**dass** die Umsetzung in einer Vorrichtung gemäß einem der Ansprüche 1 bis 9 durchgeführt wird, und dass das Zielprodukt der Umsetzung mit dem Permeat der Membrantrennstufe aus dem Flüssigkeitsumlauf ausgetragen wird.

11. Verfahren nach Anspruch 10,

**dadurch gekennzeichnet,**

**dass** der äußere Flüssigkeitsumlauf stromaufwärts vor der Membrantrennstufe ein Gemisch darstellt, welches eine flüssige Phase und eine darin dispers verteilte gasförmige Phase umfasst, wobei Volumenanteil der gasförmigen Phase zwischen null und dreißig Prozent beträgt.

12. Verfahren nach Anspruch 10 oder 11,

**dadurch gekennzeichnet,**

**dass** in der Vorrichtung Oxidationen, Epoxidierungen, Hydroformylierungen, Hydroaminierungen, Hydroaminomethylierungen, Hydrocyanierungen, Hydrocarboxyalkylierung, Aminierungen, Ammonoxydation, Oximierungen, Hydrosilylierungen, Ethoxylierungen, Propoxylierungen, Carbonylierungen, Telomerisierungen, Methathesen, Suzuki-Couplings oder Hydrierungen durchgeführt werden.

13. Verfahren nach Anspruch 12,

**dadurch gekennzeichnet,**

**dass** Verbindungen mit olefinischen Doppelbindungen durch Hydroformylierung mit Synthesegas zu Aldehyden und/oder Alkoholen umgesetzt werden.

14. Verwendung einer Vorrichtung nach einem der Ansprüche 1 bis 9 zur Durchführung homogenkatalytischer Umsetzungen, insbesondere zur Hydroformylierung.

**Claims**

1. Device for the continuous homogeneous-catalytic reaction of a liquid with a gas and optionally a further fluid, wherein the device comprises at least one reactor having a pump-driven external liquid circuit, and wherein the device comprises at least one membrane separation stage that preferably retains the homogeneous catalyst and has a molecular weight cutoff (MWCO) of from 150 g/mol to less than 1000 g/mol,

**characterized in that**

the reactor is a jet loop reactor, **in that** pump and membrane separation stage are arranged in the same external liquid circuit,

and **in that** a permeate-side free volume is provided in the membrane separation stage, which free volume is at least 0.3 dm$^3$ per m$^2$ of membrane surface.

2. Device according to Claim 1,

**characterized in that**

the pump is equipped structurally for long-term pumping of fluids which contain liquid and gaseous phases.

3. Device according to Claim 2,

**characterized in that**

the pump is a peripheral impeller pump.

4. Device according to any one of the preceding claims, **characterized by** a permeate-side gas discharge arranged downstream of the membrane separation stage.

5. Device according to any one of the preceding claims,

**characterized in that**

a heat exchanger for heating or cooling the feed or the permeate of the membrane separation stage is arranged in the external liquid circuit, in particular upstream of the membrane separation stage.

6. Device according to any one of the preceding claims,

**characterized in that**

a tubular reaction space extends in the jet loop reactor, into which tubular reaction space a jet nozzle for injecting the liquid into the reaction space and also a suction tube for extracting the gas by suction both open out, and on which is provided a baffle plate-shielded takeoff for the external liquid circuit.

7. Device according to any one of the preceding claims, **characterized by** at least one bypass conduit which is arranged in the external liquid circuit in parallel to the jet loop reactor or to the membrane separation stage.

8. Device according to any one of the preceding claims,
**characterized in that**
a multiplicity of jet loop reactors that are connectable in parallel and have a shared external liquid circuit are provided, wherein the membrane separation stage is arranged within the shared external liquid circuit.

9. Device according to Claim 8,
**characterized in that**
the membrane separation stage comprises a multiplicity of membranes that are connectable in parallel in such a manner that the entire active membrane surface area of the membrane separation stage is adjustable by connecting and disconnecting the membranes.

10. Process for the homogeneous-catalytic reaction of a liquid with a gas and optionally a further fluid,
**characterized in that**
the reaction is carried out in a device according to any one of claims 1 to 9, and **in that** the target product of the reaction is discharged from the liquid circuit together with the permeate of the membrane separation stage.

11. Process according to Claim 10,
**characterized in that**
the external liquid circuit upstream of the membrane separation stage is a mixture which comprises a liquid phase and a gaseous phase dispersely distributed therein, wherein the volume fraction of the gaseous phase is between zero and thirty percent.

12. Process according to Claim 10 or 11,
**characterized in that**
oxidations, epoxidations, hydroformylations, hydroaminations, hydroaminomethylations, hydrocyanations, hydrocarboxyalkylation, aminations, ammonoxidation, oximations, hydrosilylations, ethoxylations, propoxylations, carbonylations, telomerizations, methatheses, Suzuki couplings or hydrogenations are carried out in the device.

13. Process according to Claim 12,
**characterized in that**
compounds having olefinic double bonds are reacted with synthesis gas to form aldehydes and/or alcohols by hydroformylation.

14. Use of a device according to any one of Claims 1 to 9 for carrying out homogeneous-catalytic reactions, in particular for hydroformylation.

**Revendications**

1. Dispositif pour la mise en réaction continue sous catalyse homogène d'un liquide avec un gaz et éventuellement un autre fluide, le dispositif comprenant au moins un réacteur muni d'un circuit de liquide extérieur entraîné par une pompe, et le dispositif comprenant au moins un étage de séparation sur membrane retenant de préférence le catalyseur homogène, ayant un seuil de poids moléculaire (MWCO) de 150 g/mol à moins de 1 000 g/mol,
**caractérisé en ce que**
le réacteur est un réacteur à boucle et à jet, **en ce que** la pompe et l'étage de séparation sur membrane sont agencés dans le même circuit de liquide extérieur,
et **en ce qu'**un volume libre est prévu du côté du perméat dans l'étage de séparation sur membrane, qui est d'au moins 0,3 dm$^3$ par m$^2$ de surface de membrane.

2. Dispositif selon la revendication 1, **caractérisé en ce que** la pompe est conçue structuralement pour le pompage durable de fluides qui contiennent des phases liquides et gazeuses.

**3.** Dispositif selon la revendication 2, **caractérisé en ce que** la pompe est une pompe à roue périphérique.

**4.** Dispositif selon l'une quelconque des revendications précédentes, **caractérisé par** un déchargement de gaz agencé en aval après l'étage de séparation sur membrane du côté du perméat.

**5.** Dispositif selon l'une quelconque des revendications précédentes, **caractérisé en ce qu'**un échangeur de chaleur pour le chauffage ou le refroidissement de l'alimentation ou du perméat de l'étage de séparation sur membrane est agencé dans le circuit de liquide extérieur, notamment en amont de l'étage de séparation sur membrane.

**6.** Dispositif selon l'une quelconque des revendications précédentes, **caractérisé en ce qu'**une chambre de réaction tubulaire s'étend dans le réacteur à boucle et à jet, dans laquelle une buse à jet pour l'injection du liquide dans la chambre de réaction, et un tube d'aspiration pour l'aspiration du gaz débouchent ensemble, et sur laquelle un déchargement protégé par une plaque déflectrice est prévu pour le circuit de liquide extérieur.

**7.** Dispositif selon l'une quelconque des revendications précédentes, **caractérisé par** au moins une conduite de dérivation, qui est agencée dans le circuit de liquide extérieur en parallèle du réacteur à boucle et à jet ou de l'étage de séparation sur membrane.

**8.** Dispositif selon l'une quelconque des revendications précédentes, **caractérisé en ce qu'**une pluralité de réacteurs à boucle et à jet raccordables en parallèle, munis d'un circuit de liquide extérieur commun, est prévue, l'étage de séparation sur membrane étant agencé dans le circuit de liquide extérieur commun.

**9.** Dispositif selon la revendication 8, **caractérisé en ce que** l'étage de séparation sur membrane comprend une pluralité de membranes raccordables en parallèle, telles que la surface de membrane active totale de l'étage de séparation sur membrane puisse être ajustée par connexion ou déconnexion des membranes.

**10.** Procédé de mise en réaction sous catalyse homogène d'un liquide avec un gaz et éventuellement un autre fluide, **caractérisé en ce que**
la réaction est réalisée dans un dispositif selon l'une quelconque des revendications 1 à 9, et **en ce que** le produit cible de la réaction est déchargé du circuit de liquide avec le perméat de l'étage de séparation sur membrane.

**11.** Procédé selon la revendication 10, **caractérisé en ce que** le circuit de liquide extérieur prépare en amont de l'étage de séparation sur membrane un mélange qui comprend une phase liquide et une phase gazeuse dispersée dans celle-ci, la proportion en volume de la phase gazeuse étant comprise entre zéro et trente pour cent.

**12.** Procédé selon la revendication 10 ou 11, **caractérisé en ce que** des oxydations, des époxydations, des hydroformylations, des hydroaminations, des hydroaminométhylations, des hydrocyanations, des hydrocarboxyalkylations, des aminations, des ammonoxydations, des oximations, des hydrosilylations, des éthoxylations, des propoxylations, des carbonylations, des télomérisations, des méthathèses, des couplages de Suzuki ou des hydrogénations sont réalisées dans le dispositif.

**13.** Procédé selon la revendication 12, **caractérisé en ce que** des composés contenant des doubles liaisons oléfiniques sont mis en réaction avec un gaz de synthèse par hydroformylation pour former des aldéhydes et/ou des alcools.

**14.** Utilisation d'un dispositif selon l'une quelconque des revendications 1 à 9 pour la réalisation de réactions sous catalyse homogène, notamment pour l'hydroformylation.

Fig. 1

Fig. 2

Fig. 3

EP 2 753 421 B1

Fig. 4

15    $O_A$    13

25

26

14

h

27

$V_{Pf}$

14

28

Fig. 5

25

26

Fig. 6

27

$V_{Pf}$

EP 2 753 421 B1

Fig. 7

EP 2 753 421 B1

Fig. 8

EP 2 753 421 B1

EP 2 753 421 B1

Fig. 9

EP 2 753 421 B1

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1931472 B1
- WO 2010023018 A1
- DE 10308111
- EP 0781166 A


**In der Beschreibung aufgeführte Nicht-Patentliteratur**

- **JANSSEN, M. ; WILTING, J. ; MÜLLER, C. ; VOGT, D.** Continuous Rhodium-Catalyzed Hydroformylation of 1-Octene with Polyhedral Oligomeric Silsesquioxanes (POSS) Enlarged Triphenylphosphine. *Angewandte Chemie International Edition,* 2010, vol. 49, 7738-7741
- Bubble Columns. **P. ZEHNER ; M. KRAUSE.** Ullmann's Encyclopedia of Industrial Chemistry, Electronic Release. Wiley-VCH, 2005
- **FALBE, JÜRGEN.** New Syntheses with Carbon Monoxide. Springer Verlag, 1980
- **PRUETT, ROY L.** Hydroformylation. *Advances in Organometallic Chemistry,* 1979, vol. 17, 1-60
- **PRISKE, M. et al.** Reaction integrated separation of homogeneous catalysts in the hydroformylation of higher olefins by means of organophilic nanofiltration. *Journal of Membrane Science,* 15. September 2010, vol. 360 (1-2), 77-83
- Continuous Rhodium-Catalyzed Hydroformylation of 1-Octene with Polyhedral Oligomeric Silsesquioxanes (POSS) Enlarged Triphenylphosphine. **JANSSEN, M. ; WILTING, J. ; MÜLLER, C. ; VOGT, D.** Angewandte Chemie International Edition. 2010, vol. 49, 7738-7741
- **TOH, X.X. LOH ; K. LI ; A. BISMARCK ; A.G. LIVINGSTON.** In search of a standard method for the characterisation of organic solvent nanofiltration membranes. *J. Membr. Sci.,* 2007, vol. 291, 120-125
- Membranes. **I. CABASSO.** Encyclopedia of Polymer Sience and Technlogy. John Wiley and Sons, 1987
- *CHEMICAL ABSTRACTS,* 26741-53-7
- *CHEMICAL ABSTRACTS,* 14847-82-9